# EUROPEAN PATENT SPECIFICATION

(11) **EP 4 370 180 B1**
(45) Date of publication and mention of the grant of the patent: **10.12.2025**
(21) Application number: 22757701.2
(22) Date of filing: 07.08.2022
(51) Int. Cl.: A61M 11/00, A61K 31/352, A61M 15/00

(54) **CLOSED LOOP DRUG ADMINISTRATION SYSTEM AND METHOD**
ARZNEIMITTELVERABREICHUNGSSYSTEM UND -VERFAHREN MIT GESCHLOSSENEM KREISLAUF
SYSTÈME ET PROCÉDÉ D'ADMINISTRATION DE MÉDICAMENT EN BOUCLE FERMÉE

(30) Priority: 07.08.2021 US 202163260062 P
(43) Date of publication of application: 22.05.2024
(73) Proprietor: Cellotex Development Ltd, Holon 5885849 (IL)
(72) Inventor: SEGAL, Liat, Tel Aviv (IL); ATIAS, Asher, 1294000 Neot Golan (IL); SEGAL, Asaf, 6458750 Tel Aviv (IL); SCICLUNA, Jean Claude, Triq Birbal Balzan (MT); SEGAL, Itay, Tel Aviv (IL)
(74) Representative: Stellbrink & Partner Patentanwälte mbB
(86) International application number: PCT/IL2022/050856
(87) International publication number: WO 2023/017506

(56) References cited:
- EP-A2- 3 160 554
- CA-A1- 3 009 599
- US-A1- 2017 127 727
- US-A1- 2018 318 529

## Description

### Field

The invention lies in the field of inhalers and particularly in the field of portable inhalers, such as electronic inhalers. The goal of the present invention is to provide an electronic inhaler for generation of a dispersion comprising a medical substance. More particularly, the present invention relates to an electronic inhaler system, a method performed in such the system and corresponding use of the system.

### Introduction

Electronic inhalers are also known as electronic cigarettes, electronic vaporizers, electronic vape, e-cigarette or e-vape. An electronic inhaler is an electronic system designed for the consumption of substances without burning them directly, such as tobacco or derivatives. Electronic inhalers typically comprise a heating element and a power source, which would to heat up and vaporizer a liquid solution. Such liquids typically comprise one or more solutions comprising nicotine, flavoring substances, propylene glycol, glycerin, etc.

In past decade, electronic inhalers have become an extremely popular alternative to traditional cigarettes among tobacco consumers as well as a popular recreative device among non-smoker. Electronic inhaler devices exist with diverse designs, often resembling cigarettes, cigars or pipes. Moreover, the industry of electronic cigarette is a constantly evolving sector, constantly delivering enormous amounts of new products quickly adjusting to consumer's needs.

Electronic vapers comprise often a plurality of different component, such a mouthpiece, a cartridge, a heating element, a microprocessor, a power source and some other electronics. Electronic cigarettes are also capable of operating within a robust range of temperature reaching temperatures between 100-250 °C within a chamber, which allows to generate an aerosolized vapor, which in turn may be inhale by a user. Such aerosols are conceived to provide flavors and active substances capable of emulating a tobacco-like smoking experience.

Even though most of user of electronic inhalers have a history of regular smoking tobacco, this trend is swiftly changing, and nowadays several users of electronic inhalers are also non-tobacco smokers using electronic inhalers for consumption of other psychoactive substances, such as cannabis or its derivatives. Cannabis, inter alia, also known as marijuana, is a popular psychoactive agent obtained mainly from the Cannabis plat or synthesized used, among other, for medical or recreational purposes. Cannabis comprises a plurality of different substances with potential psychoactive effect such as the tetrahydrocannabinol (THC), cannabidiol (CBD) and other cannabinoids.

Use of medical Cannabis has been proven as efficient for treating various diseases and symptoms, and it currently a common practice in many countries with a growing number of prescriptions. Therapeutic effects of THC, CBD and other cannabinoids, as well as their interrelations is heavily researched, and its usages comprises son medical conditions such as pain, post-traumatic stress disorder (PTSD), anxiety, depression, fibromyalgia, and sleep disorders. However, irregulated consumption of cannabis might result in tolerance for cannabinoids through cannabinoid type 1 (CB1) and type 2 (CB2) receptors. Vaporization of marijuana products, also known as vaping, has become a prevalent mode of administration and is typically perceived to hold unique benefits compared to combustible administration methods, namely healthier a measurable process. Vaping of cannabis distillates encompasses additional advantages over vaporization of raw flowers, namely repeatable and measurable substances.

However, Cannabis's effect on the human body is very much personalized, depending on the actual person, tolerance, mental and body state, etc. That said for all cannabis effects such as pain management, anxiety, PTSD, diabetes, etc., most of which dependent on the body's initial state as well as the effect of the cannabis for the specific person. Moreover, actual compounds made through traditional pills do not enable adjustable consumption quantities as well as different compounds. As Cannabis has a wide variety of cannabinoids and terpenes (over 400 chemical compounds), preparation of various mixes with varying quantities is impossible using already prepared medication, such as pills.

Consumption of Cannabis is made through inhaling the substance; hence vaporization or smoking has the most immediate effect, however it is not measurable as the output of the system does not necessarily equals the input which was actually absorbed. The problem remains also when micro dosing measurable quantities. Consumption of cannabis flowers, either through smoking or vaporization is very much dependent on the flowers medical profile, which varies significantly between different crops, strains and even within the same flower. Additionally, an over consumption of cannabis might lead to tolerance of human body receptors, this in turn would reduce the effect for the medical user which in turn will consume more cannabis to compensate for the lack of significant effect.

Currently there is no scale to indicate and accurately measure the effect of the drug for a specific person on any given time. This is a result of the personalized effect of the substance for a specific person as well as other parameters mentioned above. For instance, different cannabis strains will have different effects over different people, and for a specific patient there may be variability in effect according to physical and mental state, including other materials/food consumption, current pain level, other illnesses etc.

Cannabis liquid has specific ratios of THC, CBD and other components, each molecule with its specific vaporization temperature. Upon use, the components' ratio should be stable and controlled with every inhale. Hence, heating the substance as a whole to a given temperature will not be adequate as the proportions of evaporated fumes will change along use from the original ratio within the cartridge. US 2017/127727 A1, US 2018/318529 A1, EP 3160554 A2, CA 3009599 A1 disclose various inhalation devices.

In light of the above, it is therefore an object of the present invention to overcome or at least to alleviated the shortcomings and disadvantages of the prior art. More particularly, it is an object of the present invention to provide a safer and less prompt to error system and a corresponding method for generation of a controllable combination of cannabis compounds.

In a first aspect, the present invention relates to a vaporizing system for generating a cannabis-based inhalable aerosol, the system comprising: a vaporizer comprising at least one evaporation chamber, at least one sensor configured to sense at least one biomarker of at least one user, and at least one connecting component, wherein the system may be configured to provide at least one dynamic cannabis profile for the at least one user. The invention is defined by the appended claims.

The at least one biomarker may comprise at least one of: pulse, heart rate variability (HRV), sleep time, sleep stages, user's voice, user's tone, user's rhythm of speech, eye movements (saccades and fixations), eye features (pupil size, blood vessels), electroencephalography (EEG), user's DNA-based cannabis compatibility profile, brain EEG waves; THC breath sensing; blood levels of a plurality of markers, such as sugar, insulin; and breathing pattern.

Moreover, the at least one sensor may be an external sensor. The vaporizer may comprise a body. The at least one sensor may be at least partially integrated into body of the vaporizer. The vaporizer may comprise at one least one heating chamber. The vaporizer may comprise at least one liquid chamber. The system may comprise at least one power source. The at least one power source may comprise a battery. The one of the at least one sensor may comprise an inhalation sensor. The at least one connecting component may comprise a communication port. The communication port may be a Bluetooth.

In one embodiment, system may be configured to connect to a charging dock. The system may comprise at least one indication component. The at least one indication component may comprise at least one of: indication lamp, and screen. The system may comprise at least one identification sensor comprising at least one of: tap sensor, touch sensor, and button for user control. The button for user control may comprise at least one two operational states comprising at least one of: on, off, and pre-heat. The at least one sensor may comprise a finger-print sensor.

On a further embodiment, the system may comprise an accelerometer. The system may comprise at least one feedback generator component configured to provide feedback to the at least one user. The at least one feedback generator component may be configured to generate at least one of: vibration feedback, sound feedback, and light feedback. The system may comprise at least one flow generator component configured to produce a complementary vapor using external air. The at least one flow generator component may be an external component. The vaporizer may comprise at least one cartridge comprising at least one fluid. The at least one cartridge may comprise at least one mixture of the at least one fluid. The at least one cartridge may comprise a unique identification number linked to at least one regulatory database. It should be understood that a regulatory database may be intended to define a database wherein the unique identification number may be recorded or from which may be generated, and may, inter alia, may comprise a pharmacist database, a pharmaceutical producer database. Such database may be further linked to medical database. The unique identification number may be machine readable. The unique identification number may be human readable. The system may be configured to identify the at least one cartridge via reading the unique identification number. The system may comprise a unique identification number.

In another embodiment, the at least one connecting component may be configured to connect to at least one external component. The at least one external component may comprise at least one external recording device, wherein the at least one connecting component connects to the at least one external recording device via at least one of: Bluetooth, infrared, and Wi-Fi.

The at least one external recording device may comprise at least one of: mobile phone, smart watch, smart ring and at least one external sensor such as a glucose sensor.

The at least one connecting component may be configured to bidirectional communicate with the at least one external component.

The at least one external component may comprise at least one remote modular component, wherein the at least one connecting component may be configured to bidirectionally communicate with the at least one remote modular component. The at least one remote modular component may comprise at least one of: server and cloud.

Furthermore, the system may comprise at least one data capturing component configured to capture user-related data comprising at least one of: user ID, time of use, current cartridge used (profile), inhale features, quantity used, duration of inhalation, airflow and vaporization temperature. Additionally or alternatively, the system may comprise at least one physiological biometric sensor configured to capture at least one user-related physiological biometric data comprising at least one of: exhaled breath volatile organic compounds (VOCs), body temperature, pulse and heart rate variability (HRV). Moreover, the system may comprise at least one physical biometric sensor configured to capture at least one user-related physical biometric data comprising at least one of: facial features, eye structures and finger parameters. The at least one physical biometric sensor may comprise at least one of: camera, and light detection and ranging (LiDAR) sensor. The camera may be a front facing camera. The camera may be a rear facing camera. The system may comprise at least one near field communication (NFC) component. The at least one connecting component may comprise an identification module, wherein the identification module may be configured to receive user-related biometric data from the at least one external component.

The at least one fluid may comprise at least one evaporation temperature. The at least one fluid may comprise at least two fluids comprising a first fluid and at least one second fluid. The first fluid may comprise a substance comprising an evaporation temperature A and the at least one second fluid comprise at least one evaporation temperature B. The evaporation temperature A may be different from the at least one evaporation temperature B. The first fluid may comprise a concentration A and the at least one second fluid may comprise at least one concentration B. The concentration A may be different from the at least one concentration B. The system may comprise a controlling component.

Moreover, the controlling component may be configured to control at least one of: the first evaporation temperature A, the at least one second evaporation temperature B, the first concentration A and the at least one second concentration B. The system may be configured to adjust the at least one dynamic cannabis profile based on at least one fluid-related parameter comprising at least one of: viscosity, mount in chamber, evaporation temperature, heating mechanism profile such as voltage, pulse, heating time. The at least one heating component may be configured to assume at least one pre-heating operational state, wherein when in the at least one pre-heating operational state, the at least one heating element provides at least one pre-heating temperature, wherein the at least one pre-heating temperature may comprise a temperature range for liquefying the at least one fluid, wherein the least one pre-heating temperature may be at a lower temperature than the at least one evaporation temperature. This is particularly advantageous, as it enables a more linear temperature graph during the process of actual evaporation. As the liquid becomes less viscoid during the preheat process, it has more touch points with the actual heating body in order to properly evaporate.

In one embodiment, the finger print sensor may be configured to recognize at least one pre-configured finger print of at least one user, wherein the finger print sensor may be configured to control activation and/or deactivation of the system upon recognition of the at least one pre-configured finger print of the least one user. The at least one identification module may comprise at least one authentication protocol to authenticate the at least one user, wherein the system may be activated upon authentication of the at least one user.

The at least one connecting component may be configured to execute at least of: sending user-related usage data to the at least one external component, and receiving at least one command dataset to be implemented in the system.

The controlling component may comprise at least one controlling module configured to control at least one dosage method, the at least one controlling module comprising at least one protocol of: pre-set dosing comprising pre-configured dosages set as an input from at least one of: a medical professional and a health care provider; automatic dosing comprising dosages based on at least one user-related bio-feedback; and measured dosing comprising dosages set by the at least one user.

The system may comprise a user interface configured to supply to the at least one user at least one system notification generated by the at least one feedback generator component. The user interface may comprise at least one of: LED; screen, vibration component and sound component.

In one embodiment, the system may comprise at least one data recording component configured to record at least one at least one user-related signal data comprising at least one of: spectroscopy such as pulse, heart rate variability (HRV); actigraphy such as sleep time, sleep stages; microphone recorded user's voice such as tone, rhythm of speech; mobile device camera such as eye movements (saccades and fixations), eye features (pupil size, blood vessels); electroencephalography (EEG) such as brain EEG waves; THC breath sensing; blood levels of a plurality of markers, such as sugar, insulin; and breathing pattern.

The at least one data recording component may be configured to bidirectionally communicate with the at least one external component. The at least one recording component may be configured to prompt the at least one user to grant permission for data uploading, wherein when may be permission granted, the at least one recording component may be configured to upload the at least one user-related signal data to a single data repository.

The single data repository may comprise at least one of: a remote server and a local server. The remote server may be allocated in a cloud. The at least one recording component may be configured to upload the at least one user-related signal data segmented and personalized.

Moreover, the system may comprise a safe database configured to allocate user-related data, wherein safe database may be blockchain-based may comprise at least one of: consortium blockchain, and private blockchain.

In a further embodiment, the system may be configured to generate time-dependent consolidated data. The time-dependent consolidated data may be based on at least one of: any of the data according to any of the preceding embodiments; inputted user-related personal information data; and inputted user-related medical history data.

The system may be configured to prompt inputting at least one of the user-related personal information data, and the user-related medical history data, wherein the system may be configured to prompt the inputting to at least one of: the at least one user, and the medical professional.

The system may be configured to predict at least one user-related event based on the time-dependent consolidated data.

The system may be configured to generate at least one action recommendation based on the at least one user-related event.

The system may be configured to monitor at least one user-related health status data.

The system may be configured to predict an evolution of a current user health status based one the at least one user-related health status data. The system may be configured to predict at least one future user health status based on at least one of: the at least one user-related health status data, and the evolution of the current user health status.

The system may comprise at least one processing module configured to implement at least one computer-implemented method. The at least one processing module may comprise at least one artificial intelligence (AI) assisted module configured to implement at least one analytical approach. The at least one artificial intelligence (AI) assisted module may be configured to predict at least one effect comprising at least one of: pain, anxiety and sleep disorder.

Moreover, the system may be configured to adjust the least one dynamic cannabis profile based on any of the monitoring and prediction as recited herein.

Keeping a stable, controlled and repeatable ratio throughout the evaporation process (static, averaged, dynamic) may be essential as the molecules interact with the same body system (endocannabinoid system). Evaporation of substance not according to prescribed (preconfigured) balance will affect the efficacy of the drug.

Moreover, the system may comprise at least one component of the system as recited herein integrated in the body of the vaporizer.

In one embodiment, the least one cartridge may comprise at least 2 formula compartments, and at least one insolation element, wherein the at least one insolation element is arranged between the at least 2 formula compartments. Additionally or alternatively, the at least one heating element may be arranged perpendicular to at least one of: the at least 2 formula compartments, and the at least one insolation element.

It should be understood that the system may also comprise using in addition or instead of a vaporizer, other devices that may provide a controllable dosage of cannabis-based fluids, such as a smart patch.

In a second aspect, the invention relates to a method for generating a cannabis-based inhalable aerosol, the method comprising: generating the cannabis-based inhalable aerosol from at least one fluid, receiving at least one biomarker data of at least one user to generated at least one user biomarker, and providing at least one at least one dynamic cannabis profile based for the at least one user, wherein a composition of the cannabis-based inhalable aerosol may be based on the at least one dynamic cannabis profile for the at least one user.

Moreover, the method comprises operating a system as recited herein. The method as recited herein is also suitable for controlling in addition or instead of a vaporizer, other devices that may provide a controllable dosage of cannabis-based fluids, such as a smart patch.

The at least one biomarker data may comprise at least one of: pulse, heart rate variability (HRV), sleep time, sleep stages, user's voice, user's tone, user's rhythm of speech, eye movements (saccades and fixations), eye features (pupil size, blood vessels), electroencephalography (EEG), user's DNA-based cannabis compatibility profile, brain EEG waves; THC breath sensing; blood levels of a plurality of markers, such as sugar, insulin; and breathing pattern.

The method may comprise at least partially sensing the at least one biomarker data. The method may comprise connecting the system to a charging dock. The method may comprise generating at least one indication signal via at least one indication component comprising at least one of: indication lamp, and screen. The method may comprise triggering at least one operational state comprising at least one state of: on, off, and pre-heat. The method may comprise at least one of: generating at least one feedback, and providing the at least one feedback to the at least one user. The at least one feedback may comprise at least one of: vibration feedback, sound feedback, and light feedback.

Moreover, the method may comprise linking at least one cartridge comprising a unique identification number to at least one regulatory database. The method may comprise identifying the at least one cartridge via reading the unique identification number. The method may comprise connecting the system to at least one external component. The at least one external component may comprise a mobile phone, wherein the method may comprise connecting the system to the mobile phone. The method may comprise bidirectionally communicating the system with the at least one external component. The at least one external component may comprise at least one remote modular component, wherein the method may comprise bidirectionally communicating with the at least one remote modular component.

In one embodiment, the method may comprise capturing user-related data comprising at least one of: user ID, time of use, current cartridge used (profile), inhale features, quantity used, duration of inhalation, and vaporization temperature.

Moreover, the method may comprise capturing at least one user-related physiological biometric data comprising at least one of: exhaled breath volatile organic compounds (VOCs), body temperature, pulse and heart rate variability (HRV). The method may comprise capturing at least one user-related physical biometric data comprising at least one of: facial features, eye structures and finger parameters.

The method may comprise receiving user-related biometric data from the at least one external component. Controlling a parameter of the at least one fluid, the parameter comprising at least one of: evaporation temperature, concentration of at least one of the at least one fluid.

The method may comprise controlling a first evaporation temperature A of a first fluid, at least one second evaporation temperature B of at least one second fluid, the first concentration A and the at least one second concentration B. The method may comprise adjusting the at least one dynamic cannabis profile based on at least one fluid-related parameter comprising at least one of: viscosity, amount in chamber, evaporation temperature, heating mechanism profile such as voltage, pulse, heating time. The method may comprise operating the system assuming at least one operational state. The system may comprise at least one heating component, wherein the method may comprise operating the at least one heating component to assume at least one pre-heating operational state, wherein when in the at least one pre-heating operational state, the method may comprise heating the at least one fluid to at least one pre-heating temperature, liquefying the at least one fluid, wherein the least one pre-heating temperature may be at a lower temperature than the at least one evaporation temperature.

The method may comprise recognizing at least one pre-configured finger print of at least one user, controlling activation and/or deactivation of the system upon recognition of the at least one pre-configured finger print of the least one user.

The method may comprise utilizing at least one identification module comprising at least one authentication protocol, wherein the method may comprise authenticating the at least one user, activating the system upon authentication of the at least one user.

The method according to any of the preceding method, wherein the method may comprise: sending user-related usage data to the at least one external component, and receiving at least one command dataset to be implemented in the system.

The method may comprise controlling at least one dosage method and executing at least one protocol of: pre-set dosing comprising pre-configured dosages set as an input from at least one of: a medical professional and a health care provider; automatic dosing comprising dosages based on at least one user-related bio-feedback; and measured dosing comprising dosages set by the at least one user.

The method may comprise supplying to the at least one user at least one notification. The method may comprise recording at least one user-related signal data comprising at least one of: spectroscopy such as pulse, heart rate variability (HRV); actigraphy such as sleep time, sleep stages; microphone recorded user's voice such as tone, rhythm of speech; mobile device camera such as eye movements (saccades and fixations), eye features (pupil size, blood vessels); electroencephalography (EEG) such as brain EEG waves;
THC breath sensing; blood levels of a plurality of markers, such as sugar, insulin; and breathing pattern.

The method may comprise recording the at least one user-related signal on at least one time-interval. The at least one time-interval may comprise a continuous time interval. The method may comprise triggering the recording of the at least one user-related signal upon receiving at least one activation action. The at least one activation action may comprise a user's activation action such as eye tracking.

The method may comprise bidirectionally communicating at least one data recording component with the at least one external component. The method may comprise prompting the at least one user to grant permission for data uploading, wherein when permission may be granted, the method may comprise uploading the at least the at least one user-related signal data to a single data repository. The single data repository may comprise at least one of: a remote server and a local server. The remote server may be allocated in a cloud.

The method may comprise at least one of: segmenting the at least one user-related signal data, and personalizing the at least one user-related signal data. The method may comprise generating time-dependent consolidated data. The time-dependent consolidated data may be based on any at least one of: data according to any of the preceding method embodiments, inputted user-related personal information data; and inputted user-related medical history data.

The method may comprise prompting at least one of: the at least one user, and the medical professional, to input at least one of: the user-related personal information data, and the user-related medical history data.

The method may comprise predicting at least one user-related event based on the time-dependent consolidated data.

The method may comprise generating at least one action recommendation based on the at least one user-related event.

The method may comprise monitoring at least one user-related health status data. The method may comprise predicting an evolution of a current user health status based one the at least one user-related health status data. The method may comprise predicting at least one future user health status based on at least one of: the at least one user-related health status data, and the evolution of the current user health status.

The method is a computer-implemented method. The method may comprise: operating at least one artificial intelligence (AI) assisted module, and implementing at least one analytical approach. The method may comprise predicting at least one effect comprising at least one of: pain, anxiety and sleep disorder. The method may comprise adjusting the least one dynamic cannabis profile based on any of the monitoring and prediction according to any of preceding method embodiments.

The method may comprise executing at least one computer-implementing model comprising at least one of: at least one biomarker, at least one cannabinoid-terpene ratio, and at least one crossing effect of cannabinoids on the at least one user. The method may comprise optimizing the at least one cannabinoid-terpene ratio based on the at least one dynamic cannabis profile. The method may comprise determining at least one model restriction comprising at least one of: number of vaping sessions for a given time period, and minimal consume quantity. The method may comprise training the at least one computer-implementing model based on the at least one biomarker. The training step may comprise an online-learning algorithm. The method may comprise using personal consumption data comprising at least one of: consumption times, specific compounds, and the at least one biomarker.

In one embodiment, the method may comprise: prompting the at least one user to input at least one symptom data, and generating a symptoms report based on the at least one symptom data.

In another embodiment, the method may comprise optimizing cannabis consumption for improving the at least one user's goals. The optimizing step may be based on the symptoms report.

The method may comprise: providing ongoing dosage recommendation for the at least one user, and validation of ratio of unwanted effect-cannabis, wherein the method may comprise using inputs of symptom prediction, predicted effect of cannabis consumption and current effect of cannabis consumption.

The method may comprise displaying the dosage recommendation for the at least one user to at least one of: the at least one user, at least one medical practitioner, and at least one health care provider.

The method may comprise: monitoring the at least one fluid, generating an evaporation profile for the at least one fluid, generating a viscosity profile for the at least one fluid, and generating a temperature profile for the at least one fluid. The method may comprise monitoring a contain status of the at least one cartridge. The method may comprise automatically generating a calibration protocol. The method may comprise implementing the calibration protocol. The method may comprise automatically implementing the calibration protocol. The method may comprise determining at least one baseline. The method may comprise monitoring the at least one baseline over time. The method may comprise estimating at least one compound dosage. The estimating step may comprise at least one of: pre-measuring dosages set by the system, measuring substance intake while the at least one user freely inhales, and measuring dosages intake while measuring the substance intake.

The method may comprise adjusting the at least one dynamic cannabis profile based on at least one of personal effect of cannabis consumption such as well-being, underlying conditions, tolerance, body parameters such weight, height; and personal parameters such age, gender, the at least one biomarker.

The method implementing a setup process for the at least one user, wherein the setup process may comprise: recording a base condition in at least one time measurement without consumption of cannabis, setting as baseline based on the at least one measurement without consumption of cannabis, recording value of the at least one biomarker of the at least one user, and recording user's input of current health status such as pain. It should be understood that the term base condition is intended to refer to the point in time which wellness assessment of the at least one user is made, either by feedback provided directed by the at least one user or based on the at least one biomarker data, which may suggest existing or forecasted reduced wellness of the at least one user. Post base condition assessment recommend is provide for follow up assessment, drug intake recommendation, complementary treatment or suggesting other services as medical professional consultation. The method may comprise iterative performing any of the steps according to any of the preceding method embodiments. The method may comprise comparing data generated in the iterative performing of steps with the at least one baseline. The method may comprise adjusting the at least one baseline based on subsequent measurement of user-related data according to any of the preceding method embodiments to generate an adjusted baseline. The method may comprise adjusting the at least one dynamic cannabis profile based on the adjusted baseline. The method may comprise optimizing the at least one dynamic cannabis profile based on the iterative performing of steps.

Furthermore, the system is configured to carry out the method as recited herein. Moreover, the method according comprises operating the system as recited herein to carry out the method as recited herein.

The invention also relates to the use of the system as recited herein for carrying out the method as recited herein.

Use of medical Cannabis has been proven as efficient for treating various diseases and symptoms. Usage of Cannabis for medical purposes is now common practice in many countries with a growing number of prescriptions. Therapeutic effects of THC, CBD and other cannabinoids, as well as their interrelations is heavily researched, however some of the diseases which cannabis is becoming a common practice for including: Pain, PTSD, Anxiety, Depression, Fibromyalgia, Sleep disorders. irregulated consumption of cannabis might result in tolerance for cannabinoids through CB1 and CB2 receptors.

Vaporization of marijuana products, or "vaping," has become a prevalent mode of administration and is typically perceived to hold unique benefits compared to combustible administration methods, namely healthier a measurable process. Vaping of cannabis distillates encompasses additional advantages over vaporization of raw flowers, namely repeatable and measurable substances.

Cannabis's effect on the human body is very much personalized, depending on the actual person, tolerance, mental and body state, etc. Actual compounds made through traditional pills do not enable adjustable consumption quantities as well as different compounds. As Cannabis has a wide variety of cannabinoids and terpenes (over 400 chemical compounds), preparation of various mixes with varying quantities is impossible using already prepared medication (such as pills). Consumption is made through inhaling the substance; hence vaporization or smoking has the most immediate effect, however it is not measurable as the output of the device does not necessarily equals the input which was actually absorbed. The problem remains also when micro dosing measurable quantities. Consumption of cannabis flowers, either through smoking or vaporization is very much dependent on the flowers medical profile, which varies significantly between different crops, strains and even within the same flower. Over-consumption of cannabis might lead to tolerance of human body receptors. This in turn would reduce the effect for the medical user which in turn will consume more cannabis to compensate for the lack of significant effect. Currently there is no scale to indicate and accurately measure the effect of the drug for a specific person on any given time. Cannabis molecules have different vapor temperatures; the liquid itself has different ratio of THC, CBD and others. As the ratio should be kept with every inhale, heating the substance as a whole in a given temperature will not be adequate as the proportions of evaporated fumes will be different from the original ratio within the cartridge.

Liquid cannabis vaporization device. Liquid cannabis can be prepared in varying cannabis profiles (THC, CBD, other cannabinoids, terpenes), either personalized or mass produced. As all substances are in liquid state, the mixture can contain various compounds in varying concentrations. Vaporizer device apparatus is adapted to accommodate one or more cartridges of the liquid substance. In addition to the liquid cartridge, device will include battery, inhalation sensor, battery charging dock, battery / usage indication lamp, Bluetooth device. Cartridges vary in different compounds and are uniquely identified by serial number issued by pharmacist or producing factory and are electronically read by the device. Cartridge is assigned to a specific vaporizing device to enable accurate identification and monitoring of consumption. Vaporizer device to be connected to a smart device (mobile phone, etc.) through a range of protocols including Bluetooth, Wi-Fi or any other. This protocol will be used to capture the data from the actual device to a smart device and in turn to a shared data repository (either on cloud or on premise). Vaporizing device data to capture the following features: user, time of use, current cartridge used (profile), inhale features, quantity used; duration of inhalation. Vaporizer device to have mounted sensors including: exhaled breath volatile organic compounds (VOCs), body temperature, pulse, heart rate variability. Formulas within the cartridge may contain more than one substance (molecule) with different evaporation temperatures as well as different ratios between them. Keeping the ratio static throughout the evaporation process (which can last for weeks) is essential as the molecules interact with the same body system (endocannabinoid system). Evaporation of substance not according to prescribed (preconfigured) balance will affect the efficacy of the drug. In order to keep the same evaporation ratio while using the device, cartridges may contain different heating chambers to support standardized vaporization. Those aforementioned chambers will contain different liquid formulas and will have different heating temperatures adaptable to the substance which the chamber was filled with. Pre-heat mechanism will be used in order to liquidate the formulas into liquid state at a lower temperature than evaporation. This will enable: built-in fingerprint sensor to be used as an on-off indication for the device to operate. Usage will only be possible while the pre-configured fingerprint has contact with the sensor. Vaporizer data recording to be combined with one or more additional data sources (smart watch, ring, or any other device). Recorded signals may include but not limited to: Pulse, Heart rate variability, Sleep time, Sleep stages, Patient voice (features such as tone, rhythm of speech, etc.), Eye movements (saccades and fixations), Eye features (pupil size, blood vessels), Brain EEG waves, THC breath sensing, Urine, Tremors. Uploading recorded data to a single data repository (cloud or on-premise) there it will be stored in a segmented manner and personalized where applicable. Recorded data of vaporizer apparatus as well as sensing devices will be consolidated in a time-dependent manner to allow effect measurement as well as various prediction and recommendation engines. Biomarkers are readily available as indicators for diagnosis, monitoring and prediction algorithms, as in table A below:

| Type | Topic | Subject | Link |
|---|---|---|---|
| Medical trials | Eye biofeedback for Cannabis presence | Evaluation of an Eye Tracking Sensor to Detect Cannabis Impairment | https://clinicaltrials.gov/ct2/ show/NCT03994926 |
| Medical trials | Eye biofeedback for Cannabis presence | Eye Tracking as a Biomarker of Cannabis Effects | https://clinicaltrials.gov/ct2/ show/NCT04100590 |
| | Eye biofeedback for pain sensing | Eye Movements in Response to Pain-Related Feelings in the Presence of Low and High Cognitive Loads | https://www.mdpi.com/2076 -328X/10/5/92/htm |
| Implementa tion | Evaluation of pain and drug effect | New Eye-Tracking Device from AlgometRx Targets Stimuli Reactions | https://www.practicalpainma nagement.com/rasources/ne ws-and-research/new-eye-tracking-device-algometrx-targets-stimuli-reactions |
| Implementa tion | Eye biofeedback to measure fatigue | An oculometrics-based biofeedback system to impede fatigue development during computer work: A proof-of-concept study | https://journals.plos.org/plos one/article?id=10.1371/jour nal.pone.0213704 |
| | Eye biofeedback for visual impairment diagnosis and treatment | Eye Tracking Controlled Biofeedback System for Orthoptic Diagnosis and Fusion Therapy | https://lovs.arvojournals.org /article.aspx?articleid=24023 |
| Article | General eye biofeedback | Integrating VR, Eye Tracking and Biofeedback in Research | https://insidescientific.com/ webinar/vr-eye-tracking-biofeedback-research-biopac/ |
| Article | Deep learning eye tracking using mobile device | Mobile Device Gaze Estimation with Deep Learning | https://kth.diva-portal.org/smash/get/diva2: 1391030/FULLTEXT02.pdf |
| Article | Deep learning eye tracking using mobile device | Deep Neural Networks for Low-Cost Eye Tracking | https://www.researchgate.ne t/publication/344549748 De ep Neural Networks for Lo |
| | | | w-Cost Eye_Tracking |
| Article | Deep learning eye tracking using mobile device | Hybrid Eye-Tracking on a Smartphone with CNN Feature Extraction and an Infrared 3D Model | https://www.mdpi.com/1424 -8220/20/2/543/pdf |
| Article | Smart watch biofeedback | Biofeedback in the Wild - A SmartWatch Approach | https://www.researchgate.ne t/publication/328154827_Bio feedback_in_the_Wild - _A_SmartWatch_Approach |
| Article | Smart watch biofeedback | A Critical Review of Consumer Wearables, Mobile Applications, and Equipment for Providing Biofeedback, Monitoring Stress, and Sleep in Physically Active Populations | https://www.ncbi.nlm.nih.go v/pmc/articles/PMC6031746/ |
| Article | Smart watch biofeedback | | https://spcare.bmi.com/cont ent/8/2/237 |
| Article | Smart watch biofeedback | MindfulWatch: A Smartwatch-Based System For Real-Time Respiration Monitoring During Meditation | https://www.researchgate.ne t/publication/319636955 Min dfulWatch_A_Smartwatch-Based_System_For_Real-Time_Respiration_Monitoring During_Meditation |
| Article | Voice biofeedback for pain recognition | The Fingerprints of Pain in Human Voice | https://www.openu.ac.il/lists /mediaserver documents/ac ademic/cs/TheFingerprintsof PaininHumanVoice.pdf |
| Article | Terpenes | Terpenoids and Phytocannabinoids Co-Produced in Cannabis Sativa Strains Show Specific Interaction for Cell Cytotoxic Activity | https://www.ncbi.nim.nih.go v/pmc/articles/PMC6749504/ |
| Trials | Vaporization | A Qualitative Analysis of Cannabis Vaporization among Medical Users | https://www.ncbi.nlm.nih.go v/pmc/articles/PMC6737940/ |
| Article | Terpenes | Terpenes/Terpenoids in Cannabis: Are They Important? | https://www.karger.com/Arti cle/FullText/509733 |
| Article | Sleep | Prediction of sleep duration | https://academic.oup.com/sl eep/article/42/Supplement_1 /A172/5450910 |

Goal of the training method would be to decrease total consumption of cannabis while reducing / avoiding unwanted body effects such as pain, liability to sleep, specific sleep stages, etc. Each one of the biomarkers will be assessed separately and as a group in order to better quantify the most indicative markers for each person for each disease / effect he is suffering from. The following parameters will be considered as part of the model: Biomarkers - one or more, Cannabinoids & terpenes ratio (over 400 types of molecules), Crossing effects of cannabinoids (for instance, THC and CBD). Neural networks, and specifically reinforced learning will be used in order to create the model to process all relevant inputs and provide its recommendations. Model restriction will be introduced as: The number of vaping sessions for a given time (X per hour, day, etc.), Minimal quantity possible to consume. Reinforced learning training will be based on biomarkers while the agent will serve as a recommendation engine for the user or medical doctor for the amount and time of consumption. Some of the more advanced biomarkers such as EEG, eye tracking, pupil size and more will serve as part of the model training exercise, however those biomarkers will be omitted from the actual live algorithm. The reasoning behind this is that the ongoing recording should allow as normal day-to-day life as possible. EEG and exhale recording will be mostly used during the initial user configuration.

Prediction algorithms for disease symptoms are mostly used in order to accurately predict the time of the symptom such as seizure, pain, sleep phase, etc. Dosing cannabis consumption based on ongoing measurement combined with the amount and type of cannabis composition taken in order to measure the effect of cannabis on the patient body (see "Agent - environment" illustration). Inputs of symptom prediction as well as predicted and actual effect of cannabis consumption can be used in order to provide ongoing dosage recommendations for the patient as well as validation of the ratio of unwanted effect and cannabis

Recommendations will be provided to the patient and or his medical doctor. As the model should be adapted to each user as a result of the personalized effect of cannabis (tolerance, body weight, etc.) as well as the biomarkers, an initial setup process is needed in order to achieve accurate prediction and recommendations. Setup process for a user will begin in recording his base condition without consumption of cannabis. This will serve as the ground truth for any forecast. Features of the user bio markers will be stored for prediction purposes and will cover all relevant features along with user indication of the actual effect (pain, etc.). Assuming no accurate prediction can be achieved, additional biomarkers will be recorded such as pupilar size, eye tracking (fixations, saccades), voice recognition, EEG and exhale sensing for THC and other substances. These additional features will be recorded for a given period in order to maximize the predictions made using the basic biomarkers. Once accurate prediction is achieved, setup of cannabis effect for measured dosage will be recorded in order to measure the effect of cannabis for the user. Cannabis consumption will be measured using an iterative process of increasing dosages, changing formulations, adapting number of inhales, etc. Summary of steps: Measures prior to using cannabis, Prediction of unwanted effect, Measures while performing the evaporation process., Measures X amount of time past evaporation (5 min, 10 min, 15 min, 20 min, 30 min, 60 min, etc.), Ratio between unwanted effect and cannabis effect will be established and will be used as a starting point for consumption recommendation. Iterative processes will be used in order to optimize cannabis formulation and quantities based on the best fitted ratio. Blind placebo exercise will be introduced from time to time in order to eliminate any biased effect resulting from cannabis consumption (as in any other drug).

Vaporizing process: Dynamic formulations based on smart heating chambers, Personalized recommendations of usage, Dynamic measured dosage based on recommendations, Ongoing patient tracking and monitoring, Real-time monitoring of vaporizer cartridges including locking of cartridge assignment to other vaporizer apparatus , Recording of usage (input) correlation with patient signals (biomarker - output) enables better forecasting future dosage without affecting the body's ability to develop tolerance, Optimized dosage based on past (dosage profile, dosage size), Prediction of optimal usage time (prior to actual perceivable pain) .

The approach of the present invention is particularly advantageous, as the method is suitable for controlling a vaporizer system, such as a cannabis vaporizer. Moreover, the method is also advantageous, as it allows comprising monitoring one or more physiological parameters of the at least one user during a consumption of, for example, a cannabis vapor by the at least one user, to provide monitoring results, and controlling component of the system, such as, heating of different liquid formulas by the at least one evaporation chamber, e.g., by different heating chambers of the cannabis vaporizer, based on the monitoring results, wherein the heating forms the cannabis vapor. Moreover, controlling steps of the present invention allows maintaining a ratio between a set of psychoactive substances such as cannabis components within a predefined ratio range, such as one or more cannabinoids, which may comprise CBD, THC and/or at least one terpene. The method is also advantageous, as it allows that this predefined range is personalized to the at least one user, and further allows applying a machine learning process tailored to the at least one user, wherein the method may implement controlling of the system based on the machine learning process. Moreover, the present invention is also advantageous, as it allows training the machine learning process using a dataset of multiple users, which is turned may be tailor to a given user. The machine learning process may comprise performing a reference monitoring process that comprises monitoring at least one physiological parameter of the at least one user when the user does not consume the cannabis vapor. The controlling step may comprise applying the machine learning process to predict an impact of the cannabis vapor on the user, and/or changing at least one control parameter to avoid a predicted future pain. It should be understood that the one or more physiological parameters may be obtained from a plurality of sensors, such as, of a first group of sensors, wherein the controlling may be executed using a machine learning process that was trained and/or pre-trained with the at least one physiological parameter obtained from, for instance, a second group of sensors, which may comprise more type of sensors than the first group of sensors. The second group of sensors may, for example, comprise a brain EEG wave sensor, while the first group of sensors does not comprise the brain EEG wave sensor. Alternatively, the second group of sensors may comprise a sleep sensor, while the first group of sensors does not comprise said sensor. It should be understood that the one or more physiological parameters of the at least one user may also comprise one or more sleep parameters.

The approach of the present invention is also advantageous, as the method allows identifying (liquid) formulas inserted to the different heating chambers. Moreover, the method of present invention allows, inter alia, but not limited to, at least one of: monitoring using a sub-set of sensors; training using the sub-set of sensors and an additional sub-set of sensors; sensing vapor emitted from the vaporizer; controlling responsive to the sensing; generating cannabis consumption recommendations based on the monitoring; obtaining monitoring results obtained from multiple cannabis consumption sessions; forecasting future cannabis consumption that once applied does not amount to a development of cannabis tolerance; forecasting a future cannabis consumption session of maximal duration that once applied does not induce pain; forecasting future cannabis consumption that once applied does not amount to a sleep disorder; monitoring one or more parameter of the cannabis consumption that differs from the one or more physiological parameters of the at least one user; and controlling responsive to the one or more parameter of the cannabis consumption that differs from the one or more physiological parameters of the at least one user.

The present technology is also described by the following numbered embodiments.

Below, system embodiments will be discussed. These embodiments are abbreviated by the letter "S" followed by a number. When reference is herein made to a system embodiment, those embodiments are meant.

S1. A vaporizing system for generating a cannabis-based inhalable aerosol, the system comprising:
a vaporizer comprising at least one evaporation chamber,
at least one sensor configured to sense at least one biomarker of at least one user, and
at least one connecting component,
wherein the system is configured to provide at least one dynamic cannabis profile for the at least one user.

S2. The system according to the preceding embodiment, wherein the at least one biomarker comprises at least one of: pulse, heart rate variability (HRV), sleep time, sleep stages, user's voice, user's tone, user's rhythm of speech, eye movements (saccades and fixations), eye features (pupil size, blood vessels), electroencephalography (EEG), user's DNA-based cannabis compatibility profile, brain EEG waves; THC breath sensing; blood levels of a plurality of markers, such as sugar, insulin; and breathing pattern.

S3. The system according to any of the preceding system embodiments, wherein the at least one sensor is an external sensor.

S4. The system according to any of the preceding system embodiments, wherein the vaporizer comprises a body.

S5. The system according to any of the preceding system embodiments, wherein the at least one sensor is at least partially integrated into body of the vaporizer.

S6. The system according to any of the preceding system embodiments, wherein the vaporizer comprises at one least one heating chamber.

S7. The system according to any of the preceding system embodiments, wherein the vaporizer comprises at least one liquid chamber.

S8. The system according to any of the preceding system embodiments, wherein the system comprises at least one power source.

S9. The system according to the preceding embodiment, wherein the at least one power source comprises a battery.

S10. The system according to any of the preceding system embodiments, wherein one of the at least one sensor comprises an inhalation sensor.

S11. The system according to any of the preceding system embodiments, wherein the at least one connecting component comprises a communication port.

S12. The system according to the preceding embodiment, wherein the communication port is a Bluetooth.

S13. The system according to any of the preceding system embodiments, wherein the system is configured to connect to a charging dock.

S14. The system according to any of the preceding system embodiments, wherein the system comprises at least one indication component.

S15. The system according to the preceding embodiment, wherein the at least one indication component comprises at least one of: indication lamp, and screen.

S16. The system according to any of the preceding system embodiments, wherein the system comprises at least one identification sensor comprising at least one of: tap sensor, touch sensor, and button for user control.

S17. The system according to the preceding embodiment, wherein the button for user control comprises at least one two operational states comprising at least one of: on, off, and pre-heat.

S18. The system according to any of the preceding system embodiments, wherein the at least one sensor comprises a finger-print sensor.

S19. The system according to any of the preceding system embodiments, wherein the system comprises an accelerometer.

S20. The system according to any of the preceding system embodiments, wherein the system comprises at least one feedback generator component configured to provide feedback to the at least one user.

S21. The system according to the preceding embodiment, wherein the at least one feedback generator component is configured to generate at least one of: vibration feedback, sound feedback, and light feedback.

S22. The system according any of the preceding embodiments, wherein the system comprises at least one flow generator component configured to produce a complementary vapor using external air.

S23. The system according to the preceding embodiment, wherein the at least one flow generator component is an external component.

S24. The system according to any of the preceding system embodiments, wherein the vaporizer comprises at least one cartridge comprising at least one fluid,

S25. The system according to the preceding embodiment, wherein the at least one cartridge comprises at least one mixture of the at least one fluid.

S26. The system according to any of the 2 preceding embodiments, wherein the at least one cartridge comprises a unique identification number linked to at least one regulatory database.

S27. The system according to the preceding embodiment, wherein the unique identification number is machine readable.

S28. The system according to any of the 2 preceding embodiments, wherein the unique identification number is human readable.

S29. The system according to any of the 3 preceding embodiments, wherein the system is configured to identify the at least one cartridge via reading the unique identification number.

S30. The system according to any of the preceding system embodiment, wherein the system comprises a unique identification number.

S31. The system according to any of the preceding system embodiments, wherein the at least one connecting component is configured to bidirectionally connect to at least one external component.

S32. The system according to preceding embodiment, wherein the at least one external component comprises at least one external recording device, wherein the at least one connecting component connects to the at least one external recording device via at least one of: Bluetooth, infrared, and Wi-Fi.

S33. The system according to preceding embodiment, wherein the at least one external recording device comprises at least one of: mobile phone, smart watch, smart ring and at least one external sensor such as a glucose sensor.

S34. The system according to any of the preceding system embodiments, wherein the at least one connecting component is configured to bidirectional communicate with the at least one external component.

S35. The system according to any of the preceding system embodiments and with feature of embodiment S31, wherein the at least one external component comprises at least one remote modular component, wherein the at least one connecting component is configured to bidirectionally communicate with the at least one remote modular component.

S36. The system according to the preceding embodiment, wherein the at least one remote modular component comprises at least one of: server and cloud.

S37. The system according to any of the preceding system embodiments, wherein the system comprises at least one data capturing component configured to capture user-related data comprising at least one of: user ID, time of use, current cartridge used (profile), inhale features, quantity used, duration of inhalation, airflow and vaporization temperature.

S38. The system according to any of the preceding system embodiments, wherein the system comprises at least one physiological biometric sensor configured to capture at least one user-related physiological biometric data comprising at least one of: exhaled breath volatile organic compounds (VOCs), body temperature, pulse and heart rate variability (HRV).

S39. The system according to any of the preceding system embodiments, wherein the system comprises at least one physical biometric sensor configured to capture at least one user-related physical biometric data comprising at least one of: facial features, eye structures and finger parameters.

S40. The system according to the preceding embodiment, wherein the at least one physical biometric sensor comprises at least one of: camera, and light detection and ranging (LiDAR) sensor.

S41. The system according to the preceding embodiment, wherein the camera is a front facing camera.

S42. The system according to any of the 2 preceding embodiments, wherein the camera is a rear facing camera.

S43. The system according to any of the preceding system embodiments, wherein the system comprises at least one near field communication (NFC) component.

S44. The system according to any of the preceding system embodiments, wherein the at least one connecting component comprises an identification module, wherein the identification module is configured to receive user-related biometric data from the at least one external component.

S45. The system according to any of the preceding system embodiments, wherein the at least one fluid comprises at least one evaporation temperature.

S46. The system according to any of the preceding system embodiments, wherein the at least one fluid comprises at least two fluids comprising a first fluid and at least one second fluid.

S47. The system according to the preceding embodiment, wherein the first fluid comprises a substance comprising an evaporation temperature A and the at least one second fluid comprise at least one evaporation temperature B.

S48. The system according to preceding embodiment, wherein the evaporation temperature A is different from the at least one evaporation temperature B.

S49. The system according to any of the preceding system embodiments and with feature of embodiments S45, wherein the first fluid comprises a concentration A and the at least one second fluid comprises at least one concentration B.

S50. The system according to the preceding embodiment, wherein the concentration A is different from the at least one concentration B.

S51. The system according to any of the preceding system embodiments, wherein the system comprises a controlling component.

S52. The system according to the preceding embodiment, wherein the controlling component is configured to control at least one of: the first evaporation temperature A, the at least one second evaporation temperature B, the first concentration A and the at least one second concentration B.

S53. The system according to any of the preceding system embodiments, wherein the system is configured to adjust the at least one dynamic cannabis profile based on at least one fluid-related parameter comprising at least one of: viscosity, mount in chamber, evaporation temperature, heating mechanism profile such as voltage, pulse, heating time.

S54. The system according to any of the preceding system embodiments, wherein the at least one heating component is configured to assume at least one pre-heating operational state, wherein when in the at least one pre-heating operational state, the at least one heating element provides at least one pre-heating temperature, wherein the at least one pre-heating temperature comprises a temperature range for liquefying the at least one fluid, wherein the least one pre-heating temperature is at a lower temperature than the at least one evaporation temperature.

S55. The system according to any of the preceding system embodiments and with features of embodiment S18, wherein the finger print sensor is configured to recognize at least one pre-configured finger print of at least one user, wherein the finger print sensor is configured to control activation and/or deactivation of the system upon recognition of the at least one pre-configured finger print of the least one user.

S56. The system according to any of the preceding system embodiments and with features of embodiment S44, wherein the at least one identification module comprises at least one authentication protocol to authenticate the at least one user, wherein the system is activated upon authentication of the at least one user.

S57. The system according to any of the preceding system embodiments and with features of embodiment S36 or S35, wherein the at least one connecting component is configured to execute at least of:
sending user-related usage data to the at least one external component, and
receiving at least one command dataset to be implemented in the system.

S58. The system according to any of the preceding system embodiments and with feature of embodiment S51, wherein the controlling component comprises at least one controlling module configured to control at least one dosage method, the at least one controlling module comprising at least one protocol of:
pre-set dosing comprising pre-configured dosages set as an input from at least one of: a medical professional and a health care provider;
automatic dosing comprising dosages based on at least one user-related bio-feedback; and
measured dosing comprising dosages set by the at least one user.

S59. The system according to any of the preceding system embodiments and with feature of embodiment S21, wherein the system comprises a user interface configured to supply to the at least one user at least one system notification generated by the at least one feedback generator component.

S60. The system according to the preceding embodiment, wherein the user interface comprises at least one of: LED; screen, vibration component and sound component.

S61. The system according to any of the preceding system embodiments, wherein the system comprises at least one data recording component configured to record at least one at least one user-related signal data comprising at least one of
spectroscopy such as pulse, heart rate variability (HRV);
actigraphy such as sleep time, sleep stages;
microphone recorded user's voice such as tone, rhythm of speech;
mobile device camera such as eye movements (saccades and fixations), eye features (pupil size, blood vessels);
electroencephalography (EEG) such as brain EEG waves;
THC breath sensing;
blood levels of a plurality of markers, such as sugar, insulin; and
breathing pattern.

S62. The system according to the preceding embodiment, wherein the at least one data recording component is configured to bidirectionally communicate with the at least one external component.

S63. The system according to the preceding embodiment, wherein the at least one recording component is configured to prompt the at least one user to grant permission for data uploading, wherein when is permission granted, the at least one recording component is configured to upload the at least one user-related signal data to a single data repository.

S64. The system according to the preceding embodiment, wherein the single data repository comprises at least one of: a remote server and a local server.

S65. The system according to the preceding embodiment, wherein the remote server is allocated in a cloud.

S66. The system according to any of the 3 preceding embodiments, wherein the at least one recording component is configured to upload the at least one user-related signal data segmented and personalized.

S67. The system according to any of the preceding system embodiments, wherein the system comprises a safe database configured to allocate user-related data, wherein safe database is blockchain-based comprises at least one of: consortium blockchain, and private blockchain.

S68. The system according to any of the preceding system embodiments, wherein the system is configured to generate time-dependent consolidated data.

S69. The system according to the preceding embodiment, wherein the time-dependent consolidated data is based on at least one of:
any of the data according to any of the preceding system embodiments;
inputted user-related personal information data; and
inputted user-related medical history data.

S70. The system according to the preceding embodiment, wherein the system is configured to prompt inputting at least one of the user-related personal information data, and the user-related medical history data, wherein the system is configured to prompt the inputting to at least one of: the at least one user, and the medical professional.

S71. The system according to the preceding embodiment, wherein the system is configured to predict at least one user-related event based on the time-dependent consolidated data.

S72. The system according to the preceding embodiment, wherein the system is configured to generate at least one action recommendation based on the at least one user-related event.

S73. The system according to any of the preceding system embodiments, wherein the system is configured to monitor at least one user-related health status data.

S74. The system according to the preceding embodiment, wherein the system is configured to predict an evolution of a current user health status based one the at least one user-related health status data.

S75. The system according to any of the 2 preceding embodiments, wherein the system is configured to predict at least one future user health status based on at least one of: the at least one user-related health status data, and the evolution of the current user health status.

S76. The system according to any of the preceding system embodiments, wherein the system comprises at least one processing module configured to implement at least one computer-implemented method.

S77. The system according to the preceding embodiment, wherein the at least one processing module comprises at least one artificial intelligence (AI) assisted module configured to implement at least one analytical approach.

S78. The system according to the preceding embodiment, wherein the at least one artificial intelligence (AI) assisted module is configured to predict at least one effect comprising at least one of: pain, anxiety and sleep disorder.

S79. The system according to any of the preceding system embodiments, wherein the system is configured to adjust the least one dynamic cannabis profile based on any of the monitoring and prediction according to any of embodiments S73 to S78.

S80. The system according to any of the preceding system embodiments, wherein the at least one cartridge comprises
at least 2 formula compartments, and
at least one insolation element,
wherein the at least one insolation element is arranged between the at least 2 formula compartments.

S81. The system according to the preceding embodiment, wherein the at least one heating element is arranged perpendicular to at least one of: the at least 2 formula compartments, and the at least one insolation element.

S82. The system according to any of the preceding system embodiments, wherein the system comprises at least one component according to any of the preceding system embodiment integrated in the body of the vaporizer.

Below, method embodiments will be discussed. These embodiments are abbreviated by the letter "M" followed by a number. When reference is herein made to a method embodiment, those embodiments are meant.

M1. A method for generating a cannabis-based inhalable aerosol, the method comprising:
generating the cannabis-based inhalable aerosol from at least one fluid,
receiving at least one biomarker data of at least one user to generated at least one user biomarker, and
providing at least one at least one dynamic cannabis profile based for the at least one user,
wherein a composition of the cannabis-based inhalable aerosol is based on the at least one dynamic cannabis profile for the at least one user.

M2. The method according to the preceding embodiment, wherein the method is for operating a system according to any of the preceding system embodiments.

M3. The method according to any of the 2 preceding embodiments, wherein the at least one biomarker data comprises at least one of: pulse, heart rate variability (HRV), sleep time, sleep stages, user's voice, user's tone, user's rhythm of speech, eye movements (saccades and fixations), eye features (pupil size, blood vessels), electroencephalography (EEG), user's DNA-based cannabis compatibility profile, brain EEG waves; THC breath sensing; blood levels of a plurality of markers, such as sugar, insulin; and breathing pattern.

M4. The method according to any of the 2 preceding embodiments, wherein the method comprises at least partially sensing the at least one biomarker data.

M5. The method according to any of the preceding method embodiments, wherein the method comprises connecting the system to a charging dock.

M6. The method according to any of the preceding method embodiments, wherein the method comprises generating at least one indication signal via at least one indication component comprising at least one of: indication lamp, and screen.

M7. The method according to the preceding embodiment, wherein the method comprises triggering at least one operational state comprising at least one state of: on, off, and pre-heat.

M8. The method according to any of the preceding method embodiments, wherein the method comprises at least one of
generating at least one feedback, and
providing the at least one feedback to the at least one user.

M9. The method according to the preceding embodiment, wherein the at least one feedback comprises at least one of: vibration feedback, sound feedback, and light feedback.

M10. The method according to any of the preceding method embodiments, wherein the method comprises linking at least one cartridge comprising a unique identification number to at least one regulatory database.

M11. The method according to any of the 3 preceding embodiments, wherein the method comprises identifying the at least one cartridge via reading the unique identification number.

M12. The method according to any of the preceding method embodiments, wherein the method comprises connecting the system to at least one external component.

M13. The method according to preceding embodiment, wherein the at least one external component comprises a mobile phone, wherein the method comprises connecting the system to the mobile phone.

M14. The method according to any of the preceding method embodiments, wherein the method comprises bidirectionally communicating the system with the at least one external component.

M15. The method according to any of the 3 preceding method embodiments, wherein the at least one external component comprises at least one remote modular component, wherein the method comprises bidirectionally communicating with the at least one remote modular component.

M16. The method according to any of the preceding method embodiments, wherein the method comprises capturing user-related data comprising at least one of: user ID, time of use, current cartridge used (profile), inhale features, quantity used, duration of inhalation, and vaporization temperature.

M17. The method according to any of the preceding method embodiments, wherein the method comprises capturing at least one user-related physiological biometric data comprising at least one of: exhaled breath volatile organic compounds (VOCs), body temperature, pulse and heart rate variability (HRV).

M18. The method according to any of the preceding method embodiments, wherein the method comprises capturing at least one user-related physical biometric data comprising at least one of: facial features, eye structures and finger parameters.

M19. The method according to any of the preceding method embodiments, wherein the method comprises receiving user-related biometric data from the at least one external component.

M20. The method according to any of the preceding method embodiments, wherein controlling a parameter of the at least one fluid, the parameter comprising at least one of:
evaporation temperature,
concentration of at least one of the at least one fluid.

M21. The method according to the preceding embodiment, wherein the method comprises controlling a first evaporation temperature A of a first fluid, at least one second evaporation temperature B of at least one second fluid, the first concentration A and the at least one second concentration B.

M22. The method according to any of the preceding method embodiments, wherein the method comprises adjusting the at least one dynamic cannabis profile based on at least one fluid-related parameter comprising at least one of: viscosity, amount in chamber, evaporation temperature, heating mechanism profile such as voltage, pulse, heating time.

M23. The method according to any of the preceding method embodiments, wherein the method comprises operating the system assuming at least one operational state.

M24. The method according to the preceding embodiment, wherein the system comprises at least one heating component, wherein the method comprises operating the at least one heating component to assume at least one pre-heating operational state, wherein when in the at least one pre-heating operational state, the method comprises
heating the at least one fluid to at least one pre-heating temperature,
liquefying the at least one fluid,
wherein the least one pre-heating temperature is at a lower temperature than the at least one evaporation temperature.

M25. The method according to any of the preceding method embodiments, wherein the method comprises
recognizing at least one pre-configured finger print of at least one user,
controlling activation and/or deactivation of the system upon recognition of the at least one pre-configured finger print of the least one user.

M26. The method according to any of the preceding method embodiments, wherein the method comprises utilizing at least one identification module comprising at least one authentication protocol, wherein the method comprises
authenticating the at least one user,
activating the system upon authentication of the at least one user.

M27. The method according to any of the preceding method, wherein the method comprises
sending user-related usage data to the at least one external component, and
receiving at least one command dataset to be implemented in the system.

M28. The method according to any of the preceding method embodiments, wherein the method comprises controlling at least one dosage method and executing at least one protocol of:
pre-set dosing comprising pre-configured dosages set as an input from at least one of: a medical professional and a health care provider;
automatic dosing comprising dosages based on at least one user-related bio-feedback; and
measured dosing comprising dosages set by the at least one user.

M29. The method according to any of the preceding method embodiments, wherein the method comprises supplying to the at least one user at least one notification.

M30. The method according to any of the preceding method embodiments, wherein the method comprises recording at least one user-related signal data comprising at least one of
spectroscopy such as pulse, heart rate variability (HRV);
actigraphy such as sleep time, sleep stages;
microphone recorded user's voice such as tone, rhythm of speech;
mobile device camera such as eye movements (saccades and fixations), eye features (pupil size, blood vessels);
electroencephalography (EEG) such as brain EEG waves;
THC breath sensing;
blood levels of a plurality of markers, such as sugar, insulin; and
breathing pattern.

M31. The method according to the preceding embodiment, wherein the method comprises recording the at least one user-related signal on at least one time-interval.

M32. The method according to the preceding embodiment, wherein the at least one time-interval comprises a continuous time interval.

M33. The method according to any of the 3 preceding embodiments, wherein the method comprises triggering the recording of the at least one user-related signal upon receiving at least one activation action.

M34. The method according to the preceding embodiment, wherein the at least one activation action comprises a user's activation action such as eye tracking.

M35. The method according to the preceding embodiment, wherein the method comprises bidirectionally communicating at least one data recording component with the at least one external component.

M36. The method according to the preceding embodiment, wherein the method comprises prompting the at least one user to grant permission for data uploading, wherein when permission is granted, the method comprises uploading the at least the at least one user-related signal data to a single data repository.

M37. The method according to the preceding embodiment, wherein the single data repository comprises at least one of: a remote server and a local server.

M38. The method according to the preceding embodiment, wherein the remote server is allocated in a cloud.

M39. The method according to any of the 3 preceding embodiments, wherein the method comprises at least one of
segmenting the at least one user-related signal data, and
personalizing the at least one user-related signal data.

M40. The method according to any of the preceding method embodiments, wherein the method comprises generating time-dependent consolidated data.

M41. The method according to the preceding embodiment, wherein the time-dependent consolidated data is based on any at least one of
data according to any of the preceding method embodiments,
inputted user-related personal information data; and
inputted user-related medical history data.

M42. The method according to the preceding embodiment, wherein the method comprises prompting at least one of: the at least one user, and the medical professional, to input at least one of: the user-related personal information data, and the user-related medical history data.

M43. The method according to the preceding embodiment, wherein the method comprises predicting at least one user-related event based on the time-dependent consolidated data.

M44. The method according to the preceding embodiment, wherein the method comprises generating at least one action recommendation based on the at least one user-related event.

M45. The method according to any of the preceding method embodiments, wherein the method comprises monitoring at least one user-related health status data.

M46. The method according to the preceding embodiment, wherein the method comprises predicting an evolution of a current user health status based one the at least one user-related health status data.

M47. The method according to any of the 2 preceding embodiments, wherein the method comprises predicting at least one future user health status based on at least one of: the at least one user-related health status data, and the evolution of the current user health status.

M48. The method according to any of the preceding method embodiments, wherein the method is a computer-implemented method.

M49. The method according to the preceding embodiment, wherein the method comprises
operating at least one artificial intelligence (AI) assisted module, and
implementing at least one analytical approach.

M50. The method according to the preceding embodiment, wherein the method comprises predicting at least one effect comprising at least one of: pain, anxiety and sleep disorder.

M51. The method according to any of the preceding method embodiments, wherein the method comprises adjusting the least one dynamic cannabis profile based on any of the monitoring and prediction according to any of preceding method embodiments.

M52. The method according to any of the preceding method embodiment, wherein the method comprises executing at least one computer-implementing model comprising at least one of:
at least one biomarker,
at least one cannabinoid-terpene ratio, and
at least one crossing effect of cannabinoids on the at least one user.

M53. The method according to the preceding embodiment, wherein the method comprises optimizing the at least one cannabinoid-terpene ratio based on the at least one dynamic cannabis profile.

M54. The method according to any of the preceding method embodiments, wherein the method comprises determining at least one model restriction comprising at least one of
number of vaping sessions for a given time period, and
minimal consume quantity.

M55. The method according to any of the preceding method embodiments, wherein the method comprises training the at least one computer-implementing model based on the at least one biomarker.

M56. The method according any of the preceding method embodiments, wherein the training step comprises an online-learning algorithm.

M57. The method according to any of the preceding method embodiments, wherein the method comprises using personal consumption data comprising at least one of: consumption times, specific compounds, and the at least one biomarker.

M58. The method according to any of the preceding method embodiments, wherein the method comprises
prompting the at least one user to input at least one symptom data, and
generating a symptoms report based on the at least one symptom data.

M59. The method according to any of the preceding method embodiments, wherein the method comprises optimizing cannabis consumption for improving the at least one user's goals.

M60. The method according to any of the preceding method embodiments, wherein the optimizing step is based on the symptoms report.

M61. The method according to any of the preceding method embodiments, wherein the method comprises
providing ongoing dosage recommendation for the at least one user, and
validation of ratio of unwanted effect-cannabis,
wherein the method comprises using inputs of symptom prediction, predicted effect of cannabis consumption and current effect of cannabis consumption.

M62. The method according to the preceding embodiment, wherein the method comprises displaying the dosage recommendation for the at least one user to at least one of: the at least one user, at least one medical practitioner, and at least one health care provider.

M63. The method according to any of the preceding method embodiments, wherein the method comprises
monitoring the at least one fluid,
generating an evaporation profile for the at least one fluid,
generating a viscosity profile for the at least one fluid, and
generating a temperature profile for the at least one fluid.

M64. The method according to any of the preceding method embodiments, wherein the method comprises monitoring a contain status of the at least one cartridge.

M65. The method according to any of the preceding method embodiments, wherein the method comprises automatically generating a calibration protocol.

M66. The method according to the preceding embodiment, wherein the method comprises implementing the calibration protocol.

M67. The method according to the preceding embodiment, wherein the method comprises automatically implementing the calibration protocol.

M68. The method according to any of the preceding method embodiments, wherein the method comprises determining at least one baseline.

M69. The method according to any of the preceding method embodiments, wherein the method comprises monitoring the at least one baseline over time.

M70. The method according to any of the preceding method embodiments, wherein the method comprises estimating at least one compound dosage.

M71. The method according to the preceding embodiment, wherein the estimating step comprises at least one of:
pre-measuring dosages set by the system,
measuring substance intake while the at least one user freely inhales, and
measuring dosages intake while measuring the substance intake.

M72. The method according to any of the preceding method embodiments, wherein the method comprises adjusting the at least one dynamic cannabis profile based on at least one of
personal effect of cannabis consumption such as well-being, underlying conditions, tolerance, body parameters such weight, height; and
personal parameters such age, gender, the at least one biomarker.

M73. The method according to any of the preceding method embodiments, wherein the method implementing a setup process for the at least one user, wherein the setup process comprises
recording a base condition in at least one time measurement without consumption of cannabis,
setting as baseline based on the at least one measurement without consumption of cannabis,
recording value of the at least one biomarker of the at least one user, and
recording user's input of current health status such as pain.

M74. The method according to the preceding method embodiments, wherein the method comprises iterative performing any of the steps according to any of the preceding method embodiments.

M75. The method according to any of the preceding method embodiments, wherein the method comprises comparing data generated in the iterative performing of steps with the at least one baseline.

M76. The method according to any of the preceding method embodiments, wherein the method comprises adjusting the at least one baseline based on subsequent measurement of user-related data according to any of the preceding method embodiments to generate an adjusted baseline.

M77. The method according to any of the preceding method embodiments, wherein the method comprises adjusting the at least one dynamic cannabis profile based on the adjusted baseline.

M78. The method according to any of the preceding method embodiments, wherein the method comprises optimizing the at least one dynamic cannabis profile based on the iterative performing of steps.

S83. The system according to any of the preceding system embodiments, wherein the system is configured to carry out the method according to any of the preceding method embodiments.

M79. The method according to any of the preceding method embodiment, wherein the method comprises operating the system according to any of the preceding system embodiments to carry out the method according to any of the preceding method embodiments.

U1. Use of the system according to any of the preceding system embodiments for carrying out the method according to any of the preceding method embodiments.

Below, program embodiments will be discussed. These embodiments are abbreviated by the letter "C" followed by a number. Whenever reference is herein made to "program embodiments", these embodiments are meant.

C1. A computer-implemented program comprising instructions which, when executed by a user-device, causes the user-device to carry out the method steps according to any of the preceding method embodiments.

C2. A computer-implemented program comprising instructions which, when executed by a server, causes the server to carry out the method steps according to any of the preceding method embodiments.

C3. A computer-implemented program comprising instructions which, when executed causes by a user-device, causes the user-device and a server to carry out the method steps according to any of the preceding method embodiments.

The present invention will now be described with reference to the accompanying drawings which illustrate embodiments of the invention. These embodiments should only exemplify, but not limit, the present invention.
- Fig. 1: schematically depicts an approach towards cannabis consumption as currently implemented in the art;
- Fig. 2: schematically depicts an approach for cannabis consumption accordingly to embodiment of the present invention;
- Fig. 3: schematically depicts a reinforcement learning according to embodiments of the present invention;
- Fig. 4: schematically depicts steps of a method according to embodiments of the present invention;
- Fig. 5: schematically depicts a cartridge according to embodiments of the present invention;
- Fig. 6: schematically depicts a user setup according to steps of the method according to embodiments of the present invention

It is noted that not all the drawings carry all the reference signs. Instead, in some of the drawings, some of the reference signs have been omitted for sake of brevity and simplicity of illustration. Embodiments of the present invention will now be described with reference to the accompanying drawings.

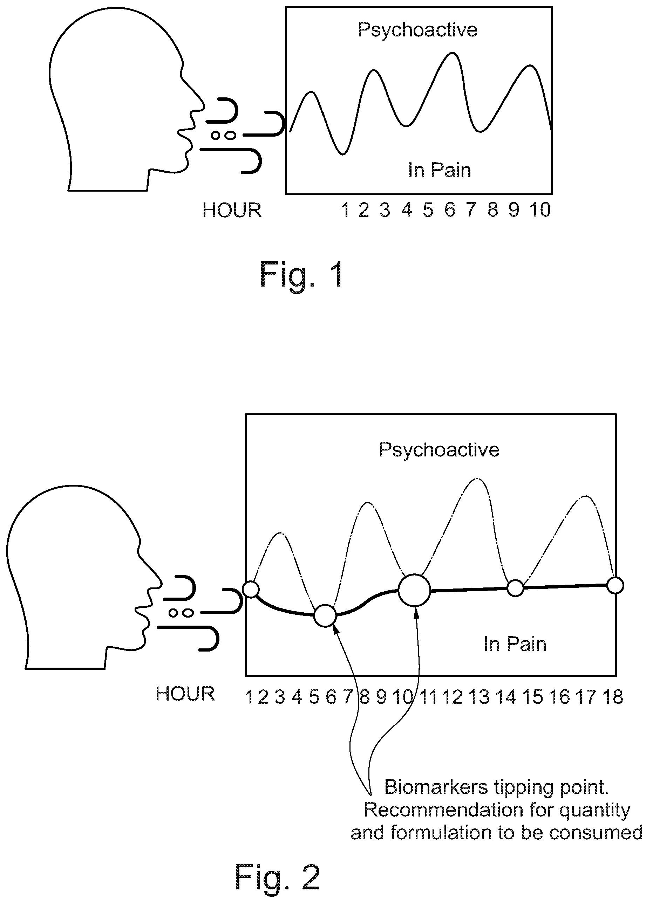

Fig. 1 schematically depicts an approach towards cannabis consumption as currently implemented in the art. In simple words, available cannabis consumption method and approaches are dependent on user awareness to pain and ability to micro dose accordingly. Put differently, currently a user is required to have a great degree of awareness of the presence of a health conditions and/or symptom, such as pain. In order to make the consumption of cannabis effective, the user is required to perform at least a micro-dosing of psychoactive substances, which is not an effective way of providing cannabis-based product to the user, as for instance, the user does necessarily possess immediate and accurate knowledge of micro dosing, combination of psychoactive, etc. In addition to this, the user is fully aware of the effects of the psychoactive over time nor of the minimum required time for substance to come into effect after consumption.

Fig. 2 schematically depicts an approach for cannabis consumption accordingly to embodiment of the present invention. In simple terms, the problems mentioned as example for Fig. 1, and other additional problems, are solve by embodiment of the present invention. Fig. 2 depicts an exemplary concept according to embodiments of the present invention, wherein the cannabis consumption is based on effect monitoring and prediction. In simple words, Fig. 2 depicts an example of the present invention, wherein biomarker data are being used at given points as tipping points. These biomarker data are used by system and method of the present invention in order to generate a recommendation for a quantity to be consumed by the user. This recommendation may be automatically implemented by the present invention. Additionally or alternative, such a recommendation may be displayed to the user, the invention may prompt the user to provide a reaction to the recommendation, such as accepting the recommendation, implementing the recommendation, rejecting the recommendation. In some embodiments of the present invention, this respond may be provided by a medical professional. Such a recommendation, may for instance require to monitor user-related data, such as symptoms, effects, and changes of the biomarker data values over time, which also be performed continuously or at a defined time interval. Such an approach is particularly advantageous to keep, for example, pain to a reduced level using an optimal amount of psychoactive in a shape a

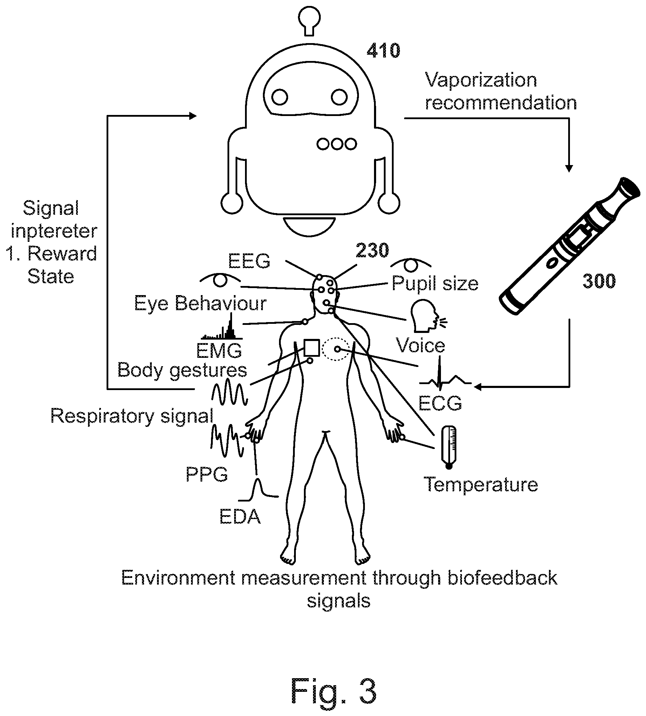

Fig. 3 schematically depicts a reinforcement learning according to embodiments of the present invention. In simple terms, the present invention comprises generating an inhalable aerosol comprising a cannabis base composition which is provided to a user 230 by means of a vaporizer 300. The present invention then captures user-related data, such as biomarker data, which is process by the system according to the embodiment of the present invention, and said data feed to the system to carry out the method according to embodiments of the present invention. In on embodiment, the data may be fed to a processing module 410, which is configured to implement at least one computer-implemented method, wherein the processing module 410 may comprise at least one artificial intelligence (AI) assisted module configured to implement at least one analytical approach. The AI assisted module may subsequently predict at least one effect on the user based on the data provided to the processing module 410 and based upon this prediction, it can, inter alia, provide vaporization recommendation, adjust a dynamic cannabis profile for the user. In one embodiment, this adjustment may be based on monitoring and prediction steps. comprising at least one of: pain, anxiety and sleep disorder. Such recommendations and/or adjustment is then transferred and implemented in the vaporizer 300. It should be understood that this process can be implemented iteratively.

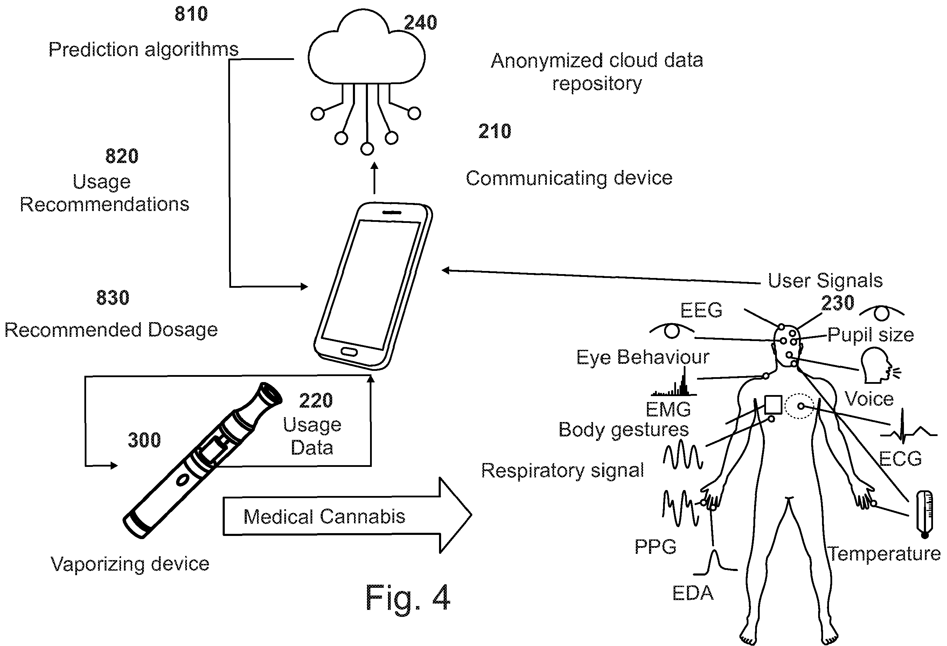

Fig. 4 schematically depicts steps of a method according to the present invention. In simple terms, a vaporizer 300 provides an inhalable aerosol comprising a cannabis-based composition, wherein the method collects user-related data 220 such as usage data from the user 230, which are sent, for instance, as signals such as user signals according to embodiments of the present invention to a device 210 such as a smart phone, wherein the method comprises transmitting the data 220, for instance, to a serve 240, wherein the method comprises implementing a plurality of steps according to embodiments of the present invention, wherein the method is a computer-implemented method comprising for instance a plurality of computer-implemented algorithms such as prediction algorithms 810. The method comprises generating usage recommendation data 820, which are transmitted, for instance, to the device 210 and implemented into the vaporizer 300 as recommended dosage 830. Such a recommended dosage is based on the user-related data, such as biomarker data. The method then comprises iteratively repeating such steps, for example, for monitoring the effect of the inhaled cannabis-based dosage. The method depicts in Fig. 4 may also comprise controlling the vaporizer 300, which may further comprise executing the method by at least one of: the vaporizer 300, the device 210, and/or a remote computer (not depicted). The method of Fig. 4 may include initializing at least one of: pre-training a machine learning process; receiving a pre-trained machine learning process; training a machine learning process after pre-training the machine; receiving a trained machine learning process; obtaining reference one or more physiological parameters of the user, and the like. It should be understood that the pre-training may use a dataset of multiple users, and may be followed by training the machine learning process to the user. Moreover, it should be understood that the training may include performing a reference monitoring process that may include, but not limited to, monitoring at least one physiological parameters of a user when the user does not consume the cannabis vapor.

Furthermore, the method may include at least one of: receiving information about previous cannabis consumption sessions of the user; receiving information about one or more physiological parameters of the user, for example, obtained during cannabis consumption; receiving information about one or more physiological parameters of the user, for example, obtained not during cannabis consumption; receiving user biometric identification information; registering the user; authenticating the user; validating the user; receiving input from the user, for example, requests from the user, preferences of the user regarding the outcome of the cannabis consumption, and the like; establishing communication between a user device and the cannabis vaporizer and/or receiving metadata (for example keys) for establishing said communication; establishing communication between a user device and a cloud computer; receiving metadata (for example keys) for establishing said communication; receiving a mapping between the content of fluid (e.g., liquid formulas) and identifiers of the cartridges that hold the liquids.

Moreover, the method may comprise implementing monitoring one or more physiological parameters of a user during a consumption of a cannabis vapor by the user, to provide monitoring results. It should be understood that the monitoring results may include the physiological parameters such as heart rate, blood pressure, and the like. In one embodiment, the monitoring may include obtaining less information about the user, in comparison to the physiological information obtained during a training of a machine learning process and/or during a pretraining of the machine learning process. In another embodiment, the monitoring may use less sensors and/or sensors of fewer types and/or sense less physiological parameters or may sense at least one physiological parameter using fewer sensors, in comparison to the training and/or the pre-training of the machine learning process. In a further embodiment, the training and/or pre-training may include at least one of the following: EEG sensed information, eye tracking information, urine information, pupil size information, user voice parameters, breath analysis, and at least one sleep parameter. The method may also comprise estimating the state of the user and/or predicting the impact of the cannabis consumption (current state or future state). The method of the present invention may comprise at least one of: evaluating eye movements to detect cannabis impairment; eye tracking for estimation of an impact of the cannabis consumption; tracking eye movements for estimating pain related feelings in the presence of high and low cognitive loads; evaluating of pain and/or cannabis consumption using, for example, AlgometRX targets stimuli reaction; tracking eye movements for estimating fatigue; and analyzing voice data for detecting pain.

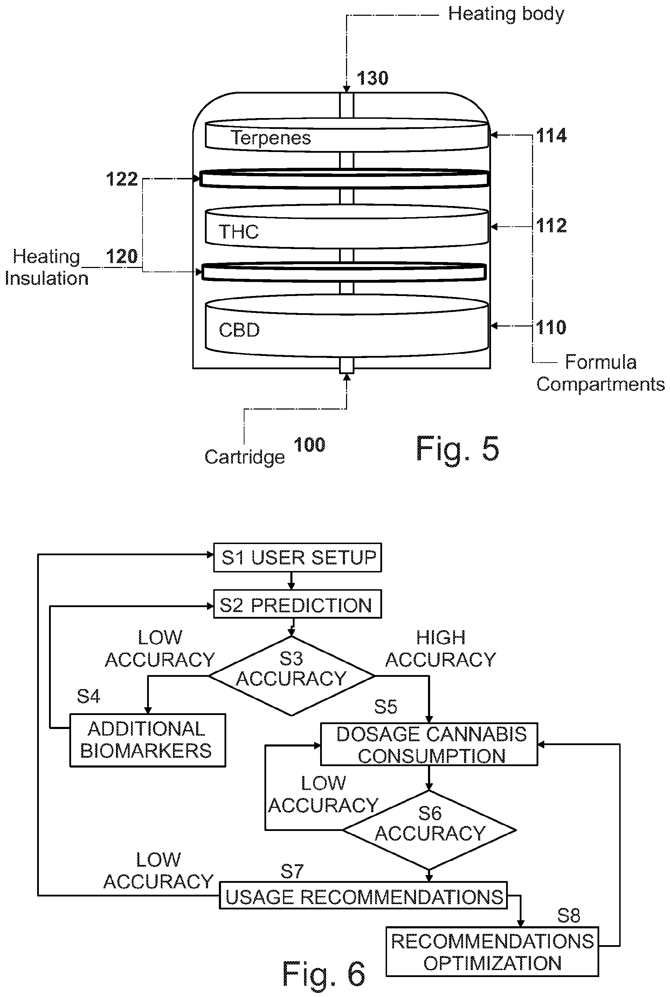

Fig. 5 schematically depicts a cartridge 100 according to embodiments of the present invention. In simple terms, the cartridge 100 comprises a heating body 130 which can also be referred to as a heating element, at least one heating insolation element 120, 122, and at least 2 formular compartments 110, 112, 114. In one embodiment, the cartridge 100 is comprised by the vaporizer 300. The cartridge 100 comprises at least one fluid and/or at least one mixture of the at least one fluid. In one embodiment, the cartridge 100 may also comprises a unique identification number (not shown) linked to a database (not shown). In one embodiment, the cartridge 100 may comprises at least 2 formula compartments 110, 112, 114, and the at least one insolation element 120, 122, wherein the at least one insolation element 120, 122 is arranged between the at least 2 formula compartments 110, 112, 114, for instance, alternating formula compartment and isolation element, for instance, 110, 120, 112, 122, 114 respectively, as depicted in Fig. 5. The heating body 130 may be arranged perpendicular to at least one of: the at least 2 formula compartments 110, 112, 114, and the at least one insolation element 120, 122. It should be understood that other arrangements are also possible (not depicted). The present invention may, for example, comprise using at least one dynamic profile for the at least one user, wherein the ratio of the at least one fluid or a mixture thereof is tailored to the at least user needs. Thus, the present invention may, for instance, implement the following parameters:

| | **Compounds** | | | **Cartridge Status** | | **Heating profile** | |
|---|---|---|---|---|---|---|---|
| Profile* | Chamber | Compound | Amount to vaporize (µ) | Current weight in cartridge (µ) | Heating temp. (Celsius) | Heating duration (seconds) | Number of pulses |
| HYBRID | A | CBD | 120 | 3,600 | 220 | 0.8 | 3 |
| | B | THC | 120 | 4500 | 160 | 0.7 | 3 |
| | C | Neutral terpene blend | 15 | 360 | 140 | 0.3 | 2 |
| Sativa - Energy | B | THC | 200 | 4380 | 160 | 0.7 | 3 |
| | D | Energy terpene blend | 30 | 360 | 140 | 0.5 | 2 |
| Indica - Pain relief | A | CBD | 200 | 3,600 | 220 | 0.8 | 3 |
| | B | THC | 120 | 4500 | 160 | 0.7 | 3 |
| | E | Relief terpene blend | 15 | 360 | 140 | 0.3 | 2 |

The system of the present invention may be configured to control a heating of different liquid formulas by different heating chambers of the cannabis vaporizer 300 and/or the cartridge 100, based on monitoring results, to form an inhalable cannabis-based aerosol. The present invention may, by means of the cartridge 100, maintain a ratio between a set of cannabis components within a predefined ratio range. Some tolerance, for example up to 0.5-20 percent, or 5-15 percent or less than 10 percent, may be allowed. The at least one fluid and/or the at least one mixture may comprise at least one cannabinoids, CBD and THC, at least one terpene, and a combination of at least one cannabinoid and at least one terpene, or any combination thereof. The cartridge 100 may further be configured to control parameter to avoid heating temperatures, initiation of heating process or ceasing of the heating process.

Fig. 6 schematically depicts a user setup S1 according to steps of the method according to embodiments of the present invention. In simple words, the Fig. 6 depicts an initial user configuration S1, which comprises carrying out the method according to embodiment of the present invention based on initial user setup S1, for which, predicting step S2 is performed. For the predicting step S2 a computer-implemented model is adapted to each user as a result of the personalized effect of cannabis such as tolerance, body weight, etc., as well as the user-related biomarker data. Hence, the computer-implemented defines an initial setup process S1, which allows achieving an accurate prediction and recommendation for the user (prediction step S3), which is assed in an accuracy step S3. Setup process for a user begins, for instance, in recording their base condition without consumption of cannabis, which would serve to determine a baseline, that can also be refer to as ground truth. It should be understood that this baseline can be used for any forecast for the user. Additional features of the user biomarkers are stored for prediction purposes and cover all relevant features along with user indication of the actual effect such as pain. This user indication can also be referred to as user feedback. If no accurate prediction S2 is achieved in S3, additional biomarkers S4 are recorded such as pupilar size, eye tracking (fixations, saccades), voice recognition, EEG and exhale sensing for THC and other substances. These additional features biomarkers S4 are recorded for a given period in order to maximize the predictions made using the basic biomarkers. If accurate prediction is achieved in S3, setup of cannabis effect for measured dosage is in order to measure the effect of cannabis for the user. This can also be referred to as dosage cannabis consumption S5. Cannabis consumption S5 is measured using an iterative process of increasing dosages, changing formulations, adapting number of inhales, etc. If enough accuracy is achieved in a second accuracy step S6, usage recommendation S7 is generated, and an optimization steps S8 for generating optimization recommendations is initiated. Generating consumption recommendations may be based on the monitoring.

While in the above, a preferred embodiment has been described with reference to the accompanying drawings, the skilled person will understand that this embodiment was provided for illustrative purpose only and should by no means be construed to limit the scope of the present invention, which is defined by the claims.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

## Claims

1. A vaporizing system for generating a cannabis-based inhalable aerosol, the system comprising:
a vaporizer comprising at least at least one evaporation chamber,
at least one sensor configured to sense at least one biomarker of at least one user, and
at least one connecting component,
wherein the system is configured to provide at least one dynamic cannabis profile for the at least one user wherein the at least one connecting component comprises an identification module, **characterized in that** the identification module is configured to receive user-related biometric data from an at least one external component.

2. The system according to the preceding claim, wherein
the at least one biomarker comprises at least one of: pulse, heart rate variability (HRV), sleep time, sleep stages, user's voice, user's tone, user's rhythm of speech, eye movements (saccades and fixations), eye features (pupil size, blood vessels), electroencephalography (EEG), user's DNA-based cannabis compatibility profile, brain EEG waves; THC breath sensing; blood levels of a plurality of markers, such as sugar, insulin; and breathing pattern;
the at least one sensor is an external sensor; and
the at least one connecting component comprises a communication port.

3. The system according to any of the preceding system claims, wherein the system comprises
at least one power source, wherein the system is configured to connect to a charging dock,
at least one indication component, wherein the at least one indication component comprises at least one of: indication lamp, and screen;
at least one identification sensor comprising at least one of: tap sensor, touch sensor, and button for user control, wherein the button for user control comprises at least one two operational states comprising at least one of: on, off, and pre-heat;
an accelerometer;
at least one feedback generator component configured to provide feedback to the user;
at least one flow generator component configured to produce a complementary vapor using external air.

4. The system according to any of the preceding system claims,
wherein a finger print sensor is configured to recognize at least one pre-configured finger print of at least one user, wherein the finger print sensor is configured to control activation and/or deactivation of the system upon recognition of the at least one pre-configured finger print of the least one user; and/or
wherein the at least one identification module comprises at least one authentication protocol to authenticate the at least one user, wherein the system is activated upon authentication of the at least one user.

5. The system according to any of the preceding system claims, wherein the at least one connecting component is configured to bidirectionally connect to at least one external component, wherein the at least one external component comprises at least one external recording device, wherein the at least one connecting component connects to the at least one external recording device via at least one of: Bluetooth, infrared, and Wi-Fi, wherein the at least one external recording device comprises at least one of: mobile phone, smart watch, smart ring and at least one external sensor such as a glucose sensor, wherein the at least one connecting component is configured to bidirectional communicate with the at least one external component, wherein the at least one external component comprises at least remote modular component, wherein the at least one connecting component is configured to bidirectionally communicate with the at least one remote modular component, wherein the at least one remote modular component comprises at least one of: server and cloud.

6. The system according to any of the preceding system claims, wherein the system comprises
at least one data capturing component configured to capture user-related data comprising at least one of: user ID, time of use, current cartridge used (profile), inhale features, quantity used, duration of inhalation, airflow and vaporization temperature;
at least one physiological biometric sensor configured to capture at least one user-related physiological biometric data comprising at least one of: exhaled breath volatile organic compounds (VOCs), body temperature, pulse and heart rate variability (HRV);
at least one physical biometric sensor configured to capture at least one user-related physical biometric data comprising at least one of: facial features, eye structures and finger parameters;
at least one near field communication (NFC) component.

7. The system according to any of the preceding system claims, wherein the at least one fluid comprises at least one evaporation temperature, wherein the at least one fluid comprises at least two fluids comprising a first fluid and at least one second fluid, wherein the first fluid comprises a substance comprising an evaporation temperature A and the at least one second fluid comprise at least one evaporation temperature B, wherein the evaporation temperature A is different from the at least one evaporation temperature B, wherein the first fluid comprises a concentration A and the at least one second fluid comprises at least one concentration B, wherein the concentration A is different from the at least one concentration B,
wherein the system comprises a controlling component, wherein the controlling component is configured to control at least one of: the first evaporation temperature A, the at least one second evaporation temperature B, the first concentration A and the at least one second concentration B,
wherein the controlling component comprises at least one controlling module configured to control at least one dosage method, the at least one controlling module comprising at least one protocol of:
pre-set dosing comprising pre-configured dosages set as an input from at least one of: a medical professional and a health care provider;
automatic dosing comprising dosages based on at least one user-related bio-feedback; and
measured dosing comprising dosages set by the at least one user.

8. The system according to any of the preceding system claims, wherein the system is configured to at least one of:
generate time-dependent consolidated data, wherein the time-dependent consolidated data is based on at least one of: any of the data according to any of the preceding system claims, inputted user-related personal information data, and inputted user-related medical history data;
prompt inputting at least one of the user-related personal information data, and the user-related medical history data, wherein the system is configured to prompt the inputting to at least one of: the at least one user, and the medical professional,
predict at least one user-related event based on the time-dependent consolidated data;
generate at least one action recommendation based on the at least one user-related event; and
monitor at least one user-related health status data and predict an evolution of a current user health status based one the at least one user-related health status data, wherein the system is configured to predict at least one future user health status based on at least one of: the at least one user-related health status data, and the evolution of the current user health status, wherein the system comprises at least one processing module configured to implement at least one computer-implemented method, wherein the at least one processing module comprises at least one artificial intelligence (AI) assisted module configured to implement at least one analytical approach, wherein the at least one artificial intelligence (AI) assisted module is configured to predict at least one effect comprising at least one of: pain, anxiety and sleep disorder, wherein the system is configured to adjust the least one dynamic cannabis profile based on based on any of: the monitoring and prediction.

9. The system according to any of the preceding claims, wherein the system comprises at least one data recording component configured to record at least one user-related signal data comprising at least one of
spectroscopy such as pulse, heart rate variability (HRV);
actigraphy such as sleep time, sleep stages;
microphone recorded user's voice such as tone, rhythm of speech;
mobile device camera such as eye movements (saccades and fixations), eye features (pupil size, blood vessels);
electroencephalography (EEG) such as brain EEG waves;
THC breath sensing;
blood levels of a plurality of markers, such as sugar, insulin; and
breathing pattern.

10. The system according to any of the preceding claims, wherein the system is configured to generate time-dependent consolidated data wherein the time-dependent consolidated data is based on at least one of:
any of the data according to any of the preceding system claims;
inputted user-related personal information data; and
inputted user-related medical history data.

11. The system according to the preceding claim, wherein the system is configured to prompt inputting at least one of the user-related personal information data, and the user-related medical history data, wherein the system is configured to prompt the inputting to at least one of: the at least one user, and the medical professional; and/or wherein the system is configured to predict at least one user-related event based on the time-dependent consolidated data.

12. The system according to the preceding claim, wherein the system is configured to generate at least one action recommendation based on the at least one user-related event.

13. The system according to any of the preceding claims, wherein the system comprises at least one processing module configured to implement at least one computer-implemented method
wherein the at least one processing module comprises at least one artificial intelligence (AI) assisted module configured to implement at least one analytical approach; and/or wherein the at least one artificial intelligence (AI) assisted module is configured to predict at least one effect comprising at least one of: pain, anxiety and sleep disorder.

14. The system according to any of the preceding claims, wherein the system is configured to adjust the least one dynamic cannabis profile based on any of the monitoring and prediction.

15. A method for generating a cannabis-based inhalable aerosol, the method comprising:
generating the cannabis-based inhalable aerosol from at least one fluid,
receiving at least one biomarker data of at least one user to generated at least one user biomarker, and
providing at least one dynamic cannabis profile based for the at least one user,
wherein a composition of the cannabis-based inhalable aerosol is based on the at least one dynamic cannabis profile for the at least one user, wherein the method is for operating a system according to any of the preceding system claims.

## Patentansprüche

1. Verdampfungssystem zum Erzeugen eines inhalierbaren Aerosols auf Cannabisbasis, wobei das System Folgendes umfasst:
einen Verdampfer, der mindestens eine Verdampfungskammer umfasst,
mindestens einen Sensor, der dazu konfiguriert ist, mindestens einen Biomarker von mindestens einem Benutzer zu erfassen, und
mindestens eine Verbindungskomponente,
wobei das System dazu konfiguriert ist, mindestens ein dynamisches Cannabisprofil für den mindestens einen Benutzer bereitzustellen, wobei die mindestens eine Verbindungskomponente ein Identifikationsmodul umfasst, **dadurch gekennzeichnet, dass**
das Identifikationsmodul dazu konfiguriert ist, benutzerbezogene biometrische Daten von mindestens einer externen Komponente zu empfangen.

2. System nach dem vorhergehenden Anspruch, wobei
der mindestens eine Biomarker mindestens eines aus Folgendem umfasst: Puls, Herzfrequenzvariabilität (HRV), Schlafzeit, Schlafstadien, Stimme des Benutzers, Tonfall des Benutzers, Sprachrhythmus des Benutzers, Augenbewegungen (Sakkaden und Fixationen), Augenmerkmale (Pupillengröße, Blutgefäße), Elektroenzephalographie (EEG), DNA-basiertes Cannabis-Kompatibilitätsprofil des Benutzers, Gehirn-EEG-Wellen; THC-Atemmessung; Blutspiegel einer Vielzahl von Markern, wie z. B. Zucker, Insulin; und Atemmuster;
der mindestens eine Sensor ein externer Sensor ist; und
die mindestens eine Verbindungskomponente einen Kommunikationsanschluss umfasst.

3. System nach einem der vorhergehenden Systemansprüche, wobei das System Folgendes umfasst:
mindestens eine Stromquelle, wobei das System dazu konfiguriert ist, mit einer Ladestation verbunden zu werden,
mindestens eine Anzeigekomponente, wobei die mindestens eine Anzeigekomponente mindestens eines der folgenden Elemente umfasst: Anzeigeleuchte und Bildschirm;
mindestens einen Identifikationssensor, der mindestens eines der folgenden Elemente umfasst: Tastsensor, Berührungssensor und Taste zur Benutzersteuerung, wobei die Taste zur Benutzersteuerung mindestens einen von zwei Betriebszuständen umfasst, die mindestens eines der folgenden Elemente umfassen: Ein, Aus und Vorheizen;
einen Beschleunigungsmesser;
mindestens eine Feedback-Erzeugungskomponente, die dazu konfiguriert ist, dem Benutzer Feedback bereitzustellen;
mindestens eine Strömungserzeugungskomponente, die dazu konfiguriert ist, einen komplementären Dampf unter Verwendung von Außenluft zu erzeugen.

4. System nach einem der vorhergehenden Systemansprüche,
wobei ein Fingerabdrucksensor dazu konfiguriert ist, mindestens einen vorkonfigurierten Fingerabdruck von mindestens einem Benutzer zu erkennen, wobei der Fingerabdrucksensor dazu konfiguriert ist, die Aktivierung und/oder Deaktivierung des Systems bei Erkennung des mindestens einen vorkonfigurierten Fingerabdrucks des mindestens einen Benutzers zu steuern; und/oder
wobei das mindestens eine Identifikationsmodul mindestens ein Authentifizierungsprotokoll umfasst, um den mindestens einen Benutzer zu authentifizieren, wobei das System bei Authentifizierung des mindestens einen Benutzers aktiviert wird.

5. System nach einem der vorhergehenden Systemansprüche, wobei die mindestens eine Verbindungskomponente dazu konfiguriert ist, sich bidirektional mit mindestens einer externen Komponente zu verbinden, wobei die mindestens eine externe Komponente mindestens eine externe Aufzeichnungsvorrichtung umfasst, wobei die mindestens eine Verbindungskomponente sich mit der mindestens einen externen Aufzeichnungsvorrichtung über mindestens eine der folgenden Komponenten verbindet: Bluetooth, Infrarot und Wi-Fi, wobei die mindestens eine externe Aufzeichnungsvorrichtung mindestens eines der folgenden Elemente umfasst: Mobiltelefon, Smartwatch, Smartring und mindestens einen externen Sensor, wie beispielsweise einen Glukosesensor, wobei die mindestens eine Verbindungskomponente dazu konfiguriert ist, bidirektional mit der mindestens einen externen Komponente zu kommunizieren, wobei die mindestens eine externe Komponente mindestens eine entfernte modulare Komponente umfasst, wobei die mindestens eine Verbindungskomponente dazu konfiguriert ist, bidirektional mit der mindestens einen entfernten modularen Komponente zu kommunizieren, wobei die mindestens eine entfernte modulare Komponente mindestens eines der folgenden Elemente umfasst: Server und Cloud.

6. System nach einem der vorhergehenden Systemansprüche, wobei das System Folgendes umfasst:
mindestens eine Datenerfassungskomponente, die dazu konfiguriert ist, benutzerbezogene Daten zu erfassen, die mindestens eines der folgenden Elemente umfassen: Benutzer-ID, Nutzungszeit, aktuell verwendete Kartusche (Profil), Inhalationsmerkmale, verwendete Menge, Dauer der Inhalation, Luftstrom und Verdampfungstemperatur;
mindestens einen physiologischen biometrischen Sensor, der dazu konfiguriert ist, mindestens eine benutzerbezogene physiologische biometrische Information zu erfassen, die mindestens eines der folgenden Elemente umfasst: ausgeatmete flüchtige organische Verbindungen (VOCs), Körpertemperatur, Puls und Herzfrequenzvariabilität (HRV);
mindestens einen physischen biometrischen Sensor, der dazu konfiguriert ist, mindestens eine benutzerbezogene physische biometrische Information zu erfassen, die mindestens eines der folgenden Merkmale umfasst: Gesichtszüge, Augenstrukturen und Fingerparameter;
mindestens eine Komponente für die Nahfeldkommunikation (NFC).

7. System nach einem der vorhergehenden Systemansprüche, wobei das mindestens eine Fluid mindestens eine Verdampfungstemperatur umfasst, wobei das mindestens eine Fluid mindestens zwei Fluide umfasst, die ein erstes Fluid und mindestens ein zweites Fluid umfassen, wobei das erste Fluid eine Substanz umfasst, die eine Verdampfungstemperatur A umfasst, und das mindestens eine zweite Fluid mindestens eine Verdampfungstemperatur B umfasst, wobei die Verdampfungstemperatur A von der mindestens einen Verdampfungstemperatur B verschieden ist, wobei das erste Fluid eine Konzentration A umfasst und das mindestens eine zweite Fluid mindestens eine Konzentration B umfasst, wobei die Konzentration A von der mindestens einen Konzentration B verschieden ist,
wobei das System eine Steuerkomponente umfasst, wobei die Steuerkomponente dazu konfiguriert ist, mindestens eines der folgenden Elemente zu steuern: die erste Verdampfungstemperatur A, die mindestens eine zweite Verdampfungstemperatur B, die erste Konzentration A und die mindestens eine zweite Konzentration B,
wobei die Steuerkomponente mindestens ein Steuermodul umfasst, das dazu konfiguriert ist, mindestens ein Dosierungsverfahren zu steuern, wobei das mindestens eine Steuermodul mindestens ein Protokoll von Folgendem umfasst:
eine voreingestellte Dosierung, die vorkonfigurierte Dosierungen umfasst, die mindestens von einem der Folgendem eingegeben wurden: einer medizinischen Fachkraft oder einem Gesundheitsdienstleister;
eine automatische Dosierung, die Dosierungen umfasst, die mindestens auf einem benutzerbezogenen Bio-Feedback basieren; und
eine gemessene Dosierung, die die von mindestens einem Benutzer eingestellten Dosierungen umfasst.

8. System nach einem der vorhergehenden Systemansprüche, wobei das System zu mindestens einem der folgenden Punkte konfiguriert ist:
Erzeugen von zeitabhängigen konsolidierten Daten, wobei die zeitabhängigen konsolidierten Daten auf mindestens einem der folgenden Elemente basieren: beliebigen Daten nach einem der vorhergehenden Systemansprüche, eingegebene benutzerbezogene Daten mit persönlichen Informationen und eingegebene benutzerbezogene medizinische Verlaufsdaten;
Auffordern zur Eingabe von mindestens einem aus den benutzerbezogenen Daten mit persönlichen Informationen und den benutzerbezogenen medizinischen Verlaufsdaten, wobei das System dazu konfiguriert ist, die Eingabeaufforderung an mindestens einen der Folgenden zu richten: den mindestens einen Benutzer und die medizinische Fachkraft,
Vorhersagen von mindestens einem benutzerbezogenen Ereignis basierend auf den zeitabhängigen konsolidierten Daten;
Erzeugen von mindestens einer Handlungsempfehlung basierend auf dem mindestens einen benutzerbezogenen Ereignis; und
Überwachen mindestens einer benutzerbezogenen Gesundheitszustandsinformation und Vorhersagen einer Entwicklung eines aktuellen Benutzergesundheitszustandes basierend auf der mindestens einen benutzerbezogenen Gesundheitszustandsinformation, wobei das System dazu konfiguriert ist, mindestens einen zukünftigen Benutzergesundheitszustand basierend auf mindestens einem der folgenden Punkte vorherzusagen: der mindestens einen benutzerbezogenen Gesundheitszustandsinformation und der Entwicklung des aktuellen Gesundheitszustands des Benutzers, wobei das System mindestens ein Verarbeitungsmodul umfasst, das dazu konfiguriert ist, mindestens ein computerimplementiertes Verfahren zu implementieren, wobei das mindestens eine Verarbeitungsmodul mindestens ein durch künstliche Intelligenz (KI) unterstütztes Modul umfasst, das dazu konfiguriert ist, mindestens einen analytischen Ansatz zu implementieren, wobei das mindestens eine durch künstliche Intelligenz (KI) unterstützte Modul dazu konfiguriert ist, mindestens einen Effekt vorherzusagen, der mindestens eines der folgenden Elemente umfasst: Schmerz, Angst und Schlafstörung, wobei das System dazu konfiguriert ist, das mindestens eine dynamische Cannabisprofil basierend auf einem der folgenden Punkte anzupassen: der Überwachung und der Vorhersage.

9. System nach einem der vorhergehenden Ansprüche, wobei das System mindestens eine Datenaufzeichnungskomponente umfasst, die dazu konfiguriert ist, mindestens eine benutzerbezogene Signalinformation aufzuzeichnen, die mindestens eines der folgenden Elemente umfasst:
Spektroskopie wie Puls, Herzfrequenzvariabilität (HRV);
Aktigraphie wie Schlafdauer, Schlafstadien;
die durch ein Mikrofon aufgezeichnete Stimme des Benutzers, z. B. den Tonfall und den Sprachrhythmus;
Kamera der mobilen Vorrichtung, wie z. B. Augenbewegungen (Sakkaden und Fixierungen), Augenmerkmale (Pupillengröße, Blutgefäße);
Elektroenzephalographie (EEG) wie Gehirn-EEG-Wellen;
THC-Atemmessung;
Blutspiegel einer Vielzahl von Markern, wie z. B. Zucker, Insulin; und
Atemmuster.

10. System nach einem der vorhergehenden Ansprüche, wobei das System dazu konfiguriert ist, zeitabhängige konsolidierte Daten zu erzeugen, wobei die zeitabhängigen konsolidierten Daten auf mindestens einem der folgenden Punkte basieren:
beliebigen Daten nach einem der vorhergehenden Systemansprüche;
eingegebenen benutzerbezogenen Daten mit persönlichen Informationen; und
eingegebenen benutzerbezogenen medizinischen Verlaufsdaten.

11. System nach dem vorhergehenden Anspruch, wobei das System dazu konfiguriert ist, die Eingabe von mindestens einem aus den benutzerbezogenen Daten mit persönlichen Informationen und den benutzerbezogenen medizinischen Verlaufsdaten aufzufordern, wobei das System dazu konfiguriert ist, die Eingabeaufforderung an mindestens einen der Folgenden zu richten: den mindestens einen Benutzer und die medizinische Fachkraft, wobei das System dazu konfiguriert ist, mindestens ein benutzerbezogenes Ereignis basierend auf den zeitabhängigen konsolidierten Daten vorherzusagen.

12. System nach dem vorhergehenden Anspruch, wobei das System dazu konfiguriert ist, mindestens eine Handlungsempfehlung basierend auf dem mindestens einen benutzerbezogenen Ereignis zu erzeugen.

13. System nach einem der vorhergehenden Ansprüche, wobei das System mindestens ein Verarbeitungsmodul umfasst, das dazu konfiguriert ist, mindestens ein computerimplementiertes Verfahren zu implementieren
wobei das mindestens eine Verarbeitungsmodul mindestens ein durch künstliche Intelligenz (KI) unterstütztes Modul umfasst, das dazu konfiguriert ist, mindestens einen analytischen Ansatz zu implementieren; und/oder
wobei das mindestens eine durch künstliche Intelligenz (KI) unterstützte Modul dazu konfiguriert ist, mindestens einen Effekt vorherzusagen, der mindestens eines der folgenden Elemente umfasst: Schmerz, Angst und Schlafstörung.

14. System nach einem der vorhergehenden Ansprüche, wobei das System dazu konfiguriert ist, das mindestens eine dynamische Cannabisprofil basierend auf einem aus der Überwachung und der Vorhersage anzupassen.

15. Verfahren zum Erzeugen eines inhalierbaren Aerosols auf Cannabisbasis, wobei das Verfahren umfasst:
Erzeugen des inhalierbaren Aerosols auf Cannabisbasis aus mindestens einem Fluid,
Empfangen mindestens einer Biomarkerinformation von mindestens einem Benutzer, um mindestens einen Benutzer-Biomarker zu erzeugen, und
Bereitstellen mindestens eines dynamischen Cannabisprofils für den mindestens einen Benutzer,
wobei eine Zusammensetzung des inhalierbaren Aerosols auf Cannabisbasis auf dem mindestens einen dynamischen Cannabisprofil für den mindestens einen Benutzer basiert, wobei das Verfahren zum Betreiben eines Systems nach einem der vorhergehenden Systemansprüche dient.

## Revendications

1. - Système de vaporisation pour générer un aérosol inhalable à base de cannabis, le système comprenant :
un vaporisateur comprenant au moins une chambre d'évaporation ;
au moins un capteur configuré pour détecter au moins un biomarqueur d'au moins un utilisateur ; et
au moins un composant de connexion,
le système étant configuré pour fournir au moins un profil de cannabis dynamique pour ledit au moins un utilisateur, ledit au moins un composant de connexion comprenant un module d'identification, **caractérisé par le fait que** le module d'identification est configuré pour recevoir des données biométriques relatives à l'utilisateur en provenance d'au moins un composant externe.

2. - Système selon la revendication précédente, dans lequel
ledit au moins un biomarqueur comprend au moins l'un parmi: le pouls, la variabilité de la fréquence cardiaque (VFC), le temps de sommeil, les stades du sommeil, la voix de l'utilisateur, le ton de l'utilisateur, le rythme de la parole de l'utilisateur, les mouvements oculaires (saccades et fixations), les caractéristiques des yeux (taille de la pupille, vaisseaux sanguins), l'électroencéphalographie (EEG), le profil de compatibilité avec le cannabis basé sur l'ADN de l'utilisateur, les ondes EEG cérébrales, la détection du THC dans l'haleine, les taux sanguins d'une pluralité de marqueurs, tels que le sucre, l'insuline, et le rythme respiratoire ;
ledit au moins un capteur est un capteur externe ; et
ledit au moins un composant de connexion comprend un port de communication.

3. - Système selon l'une quelconque des revendications précédentes, dans lequel le système comprend
au moins une source d'alimentation, le système étant configuré pour se connecter à une station de charge ;
au moins un composant d'indication, ledit au moins un composant d'indication comprenant au moins l'un parmi : une lampe d'indication et un écran ;
au moins un capteur d'identification comprenant au moins l'un parmi : un capteur de frappe, un capteur tactile et un bouton de commande par l'utilisateur, le bouton de commande par l'utilisateur comprenant au moins un ou deux états opérationnels comprenant au moins l'un parmi : marche, arrêt et préchauffage ;
un accéléromètre ;
au moins un composant générateur de retour d'information configuré pour fournir un retour d'information à l'utilisateur ;
au moins un composant générateur de flux configuré pour produire une vapeur complémentaire à l'aide d'air extérieur.

4. - Système selon l'une quelconque des revendications précédentes,
dans lequel un capteur d'empreinte digitale est configuré pour reconnaître au moins une empreinte digitale préconfigurée d'au moins un utilisateur, le capteur d'empreinte digitale étant configuré pour commander l'activation et/ou la désactivation du système lors de la reconnaissance de ladite au moins une empreinte digitale préconfigurée dudit moins un utilisateur ; et/ou
dans lequel ledit au moins un module d'identification comprend au moins un protocole d'authentification pour authentifier ledit au moins un utilisateur, le système étant activé lors de l'authentification dudit au moins un utilisateur.

5. - Système selon l'une quelconque des revendications précédentes, dans lequel ledit au moins un composant de connexion est configuré pour se connecter de manière bidirectionnelle à au moins un composant externe, dans lequel ledit au moins un composant externe comprend au moins un dispositif d'enregistrement externe, dans lequel ledit au moins un composant de connexion se connecte audit au moins un dispositif d'enregistrement externe par l'intermédiaire d'au moins l'un parmi : Bluetooth, infrarouge et Wi-Fi, dans lequel ledit au moins un dispositif d'enregistrement externe comprend au moins l'un parmi : téléphone mobile, montre intelligente, bague intelligente et au moins un capteur externe tel qu'un capteur de glucose, dans lequel ledit au moins un composant de connexion est configuré pour communiquer de manière bidirectionnelle avec ledit au moins un composant externe, dans lequel ledit au moins un composant externe comprend au moins un composant modulaire distant, dans lequel ledit au moins un composant de connexion est configuré pour communiquer de manière bidirectionnelle avec ledit au moins un composant modulaire distant, dans lequel ledit au moins un composant modulaire distant comprend au moins l'un parmi : un serveur et un nuage.

6. - Système selon l'une quelconque des revendications précédentes, dans lequel le système comprend
au moins un composant de capture de données configuré pour capturer des données relatives à l'utilisateur comprenant au moins l'un parmi : l'identifiant (ID) de l'utilisateur, le moment d'utilisation, la cartouche courante utilisée (profil), les caractéristiques de l'inhalation, la quantité utilisée, la durée de l'inhalation, le débit d'air et la température de vaporisation ;
au moins un capteur biométrique physiologique configuré pour capturer au moins une donnée biométrique physiologique liée à l'utilisateur, comprenant au moins l'un parmi : composés organiques volatils (COV) de l'air expiré, température corporelle, pouls et variabilité de la fréquence cardiaque (VFC) ;
au moins un capteur biométrique physique configuré pour capturer au moins une donnée biométrique physique liée à l'utilisateur, comprenant au moins l'un parmi : traits du visage, structures des yeux et paramètres des doigts ;
au moins un composant de communication en champ proche (NFC).

7. - Système selon l'une quelconque des revendications précédentes, dans lequel ledit au moins un fluide comprend au moins une température d'évaporation, dans lequel ledit au moins un fluide comprend au moins deux fluides comprenant un premier fluide et au moins un second fluide, dans lequel le premier fluide comprend une substance comprenant une température d'évaporation A et ledit au moins un second fluide comprend au moins une température d'évaporation B, dans lequel la température d'évaporation A est différente de ladite au moins une température d'évaporation B, dans lequel le premier fluide comprend une concentration A et ledit au moins un second fluide comprend au moins une concentration B, dans lequel la concentration A est différente de ladite au moins une concentration B,
dans lequel le système comprend un composant de commande, dans lequel le composant de commande est configuré pour commander au moins l'une parmi : la première température d'évaporation A, ladite au moins une seconde température d'évaporation B, la première concentration A et ladite au moins une seconde concentration B,
dans lequel le composant de commande comprend au moins un module de commande configuré pour commander au moins un procédé de dosage, ledit au moins un module de commande comprenant au moins un protocole de :
dosage préétabli comprenant des dosages préconfigurés établis comme entrée par au moins l'un parmi un professionnel de santé et un prestataire de soins de santé ;
dosage automatique comprenant des dosages basés sur au moins un bio-retour d'information relatif à l'utilisateur ; et
dosage mesuré comprenant des dosages définis par ledit au moins un utilisateur.

8. - Système selon l'une quelconque des revendications précédentes, dans lequel le système est configuré pour au moins l'un parmi :
générer des données consolidées dépendantes du temps, les données consolidées dépendantes du temps étant basées sur au moins l'un parmi : toutes données selon l'une quelconque des revendications de système précédentes, des données saisies d'informations personnelles relatives à l'utilisateur et des données saisies relatives aux antécédents médicaux de l'utilisateur ;
inviter à la saisie d'au moins l'un parmi les données d'informations personnelles relatives à l'utilisateur et les données relatives aux antécédents médicaux de l'utilisateur, le système étant configuré pour inviter à la saisie au moins l'un parmi : ledit au moins un utilisateur et le professionnel de santé ;
prédire au moins un événement relatif à l'utilisateur sur la base des données consolidées dépendantes du temps ;
générer au moins une recommandation d'action sur la base dudit au moins un événement relatif à l'utilisateur ; et
surveiller au moins une donnée relative à l'état de santé de l'utilisateur et prédire une évolution d'un état de santé actuel de l'utilisateur sur la base de l'au moins une donnée relative à l'état de santé de l'utilisateur, le système étant configuré pour prédire au moins un état de santé futur de l'utilisateur sur la base d'au moins l'une parmi : ladite au moins une donnée relative à l'état de santé de l'utilisateur et l'évolution de l'état de santé actuel de l'utilisateur, le système comprenant au moins un module de traitement configuré pour mettre en œuvre au moins un procédé mis en œuvre par ordinateur, ledit au moins un module de traitement comprenant au moins un module assisté par intelligence artificielle (IA) configuré pour mettre en œuvre au moins une approche analytique, ledit au moins un module assisté par intelligence artificielle (IA) étant configuré pour prédire au moins un effet comprenant au moins l'un parmi : la douleur, l'anxiété et les troubles du sommeil, le système étant configuré pour ajuster ledit au moins un profil de cannabis dynamique sur la base de l'un quelconque parmi : la surveillance et la prédiction.

9. - Système selon l'une quelconque des revendications précédentes, dans lequel le système comprend au moins un composant d'enregistrement de données configuré pour enregistrer au moins une donnée de signal relative à l'utilisateur comprenant au moins l'un parmi
la spectroscopie telle que le pouls, la variabilité de la fréquence cardiaque (VFC) ;
l'actigraphie, comme le temps de sommeil, les stades de sommeil ;
la voix de l'utilisateur enregistrée par microphone, telle que le ton, le rythme de la parole ;
une caméra de dispositif mobile, telle que mouvements oculaires (saccades et fixations), caractéristiques des yeux (taille de la pupille, vaisseaux sanguins) ;
l'électroencéphalographie (EEG) telle qu'ondes EEG cérébrales ;
la détection du THC dans l'haleine ;
les taux sanguins d'une pluralité de marqueurs, tels que le sucre, l'insuline ; et
le rythme respiratoire.

10. - Système selon l'une quelconque des revendications précédentes, dans lequel le système est configuré pour générer des données consolidées dépendantes du temps, les données consolidées dépendantes du temps étant basées sur au moins l'un parmi :
toutes données selon l'une quelconque des revendications de système précédentes ;
des données saisies d'informations personnelles relatives à l'utilisateur ; et
des données saisies relatives aux antécédents médicaux de l'utilisateur.

11. - Système selon la revendication précédente, dans lequel le système est configuré pour inviter à la saisie d'au moins l'un parmi les données personnelles relatives à l'utilisateur et les données relatives aux antécédents médicaux de l'utilisateur, dans lequel le système est configuré pour inviter à la saisie au moins l'un parmi : ledit au moins un utilisateur et le professionnel de santé ; et/ou
dans lequel le système est configuré pour prédire au moins un événement relatif à l'utilisateur sur la base des données consolidées dépendantes du temps.

12. - Système selon la revendication précédente, dans lequel le système est configuré pour générer au moins une recommandation d'action sur la base dudit au moins un événement relatif à l'utilisateur.

13. - Système selon l'une quelconque des revendications précédentes, dans lequel le système comprend au moins un module de traitement configuré pour mettre en œuvre au moins un procédé mis en œuvre par ordinateur,
dans lequel ledit au moins un module de traitement comprend au moins un module assisté par intelligence artificielle (IA) configuré pour mettre en œuvre au moins une approche analytique ; et/ou
dans lequel ledit au moins un module assisté par intelligence artificielle (IA) est configuré pour prédire au moins un effet comprenant au moins l'un parmi : la douleur, l'anxiété et les troubles du sommeil.

14. - Système selon l'une quelconque des revendications précédentes, dans lequel le système est configuré pour ajuster ledit au moins un profil de cannabis dynamique sur la base de l'une quelconque parmi la surveillance et la prédiction.

15. - Procédé de génération d'un aérosol inhalable à base de cannabis, le procédé comprenant :
générer l'aérosol inhalable à base de cannabis à partir d'au moins un fluide ;
recevoir au moins une donnée de biomarqueur d'au moins un utilisateur pour générer au moins un biomarqueur d'utilisateur ; et
fournir au moins un profil de cannabis dynamique basé pour ledit au moins un utilisateur,
dans lequel une composition de l'aérosol inhalable à base de cannabis est basée sur ledit au moins un profil de cannabis dynamique pour ledit au moins un utilisateur, le procédé étant destiné au fonctionnement d'un système selon l'une quelconque des revendications précédentes.
